# EUROPEAN PATENT SPECIFICATION

(11) **EP 2 784 061 B1**
(45) Date of publication and mention of the grant of the patent: **07.11.2018**
(21) Application number: 12851308.2
(22) Date of filing: 26.11.2012
(51) Int. Cl.: C07C 251/86, C07D 271/107, C07D 233/54, C07D 333/02, A61K 31/166, A61K 31/4164, A61K 31/381, A61P 35/02

(54) **ACYL-HYDRAZONE AND OXADIAZOLE COMPOUNDS, PHARMACEUTICAL COMPOSITIONS CONTAINING THE SAME AND USES THEREOF**
ACYLHYDRAZON- UND OXADIAZOLVERBINDUNGEN, DIESE ENTHALTENDE PHARMAZEUTISCHE ZUSAMMENSETZUNGEN UND VERWENDUNGEN DAVON
COMPOSÉS ACYL-HYDRAZONES ET OXADIAZOLS, COMPOSITIONS PHARMACEUTIQUES LES COMPRENANT ET UTILISATIONS CORRESPONDANTES

(30) Priority: 25.11.2011 BR PI1107312
(43) Date of publication of application: 01.10.2014
(73) Proprietor: Universidade Federal De Santa Catarina, 88040-900 Florianópolis SC (BR); Centro Infantil de Investigaçóes Hematológicas Dr. Domingos A. Boldrini, CEP 13083-210, Campinas, São Paulo (BR)
(72) Inventor: NUNES, Ricardo José, Pantanal, Florianópolis CEP-88040-270 Santa Catarina (BR); MASCARELLO, Alessandra, Trindade, Florianópolis CEP-88040-480 Santa Catarina (BR); YUNES, Rosendo Augusto, Cacupé Florianópolis, CEP-88050-005 (BR); STUMPF, Taisa Regina, Pantanal Florianópolis CEP-88040-320 Santa Catarina (BR); LEAL, Paulo Cesar, Ponte do Imaruim, Palhoça CEP-13085-420 Santa Catarina (BR); YUNES, José Andres, CEP-13085-420 Campinas, São Paulo (BR); PEREIRA DE SOUZA MELO, Carolina, Itabirito, CEP-35450-000 Minas Gerais (BR); CANEVAROLO, Rafael Renatino, Campinas CEP-13084-791 São Paulo (BR); DOMENEGHINI CHIARADIA, Louise, Pantanal, Florianópolis CEP-88040-000 Santa Catarina (BR); BORTOLINI SILVEIRA, André, Campinas CEP-13085-260 São Paulo (BR); LARANJEIRA, Angelo Brunelli Albertoni, 13073-035, Campinas/SP (BR)
(74) Representative: Potter Clarkson LLP
(86) International application number: PCT/BR2012/000480
(87) International publication number: WO 2013/075199

(56) References cited:
- CN-A- 1 824 657
- US-A1- 2011 189 306
- DATABASE Pubchem Compound [Online] NCBI; 9 July 2005 (2005-07-09), XP002738158, Database accession no. CID 847506
- DATABASE Pubchem Compound [Online] NCBI; 26 March 2005 (2005-03-26), XP002738159, Database accession no. CID 331793
- DATABASE Pubchem Compound [Online] NCBI; 26 March 2005 (2005-03-26), XP002738160, Database accession no. CID 285812
- DATABASE Pubchem Compound [Online] NCBI; 9 July 2005 (2005-07-09), XP002738161, Database accession no. CID 830735
- DATABASE Pubchem Compound [Online] NCBI; 16 July 2005 (2005-07-16), XP002738162, Database accession no. CID 2693709
- DATABASE Pubchem Compound [Online] NCBI; 16 September 2005 (2005-09-16), XP002738163, Database accession no. CID 4650503
- MAZZONE, G.; BONINA, F.; PUGLISI, G.; PANICO, A. M.; ARRIGO REINA, R.: "2,5-Diaryl-substituted 1,3,4-oxadiazoles: synthesis and preliminary pharmacological research", FARMACO, vol. 39, no. 5, 1984, pages 414-420, XP009183508,
- LAUREN LEE ET AL: "Design, Synthesis, and Biological Evaluations of 2,5-Diaryl-2,3-dihydro-1,3,4-oxadiazoline Analogs of Combretastatin-A4", JOURNAL OF MEDICINAL CHEMISTRY, vol. 53, no. 1, 14 January 2010 (2010-01-14), pages 325-334, XP055180271, ISSN: 0022-2623, DOI: 10.1021/jm901268n
- SHEHATA, I. A.; NASR, M. N.; EL-SUBBAGH, H. I.; GINEINAH, M. M.; KHEIRA, S. M.: "Synthesis and biological testing of certain 1,3,4-oxadiazole and 1,2,4-triazole derivatives as potential antimicrobial agents", SCIENTIA PHARMACEUTICA, vol. 64, no. 2, 1996, pages 133-143, XP009183506,
- THAS MOREIRA OSRIO ET AL: "Antibacterial activity of chalcones, hydrazones and oxadiazoles against methicillin-resistant", BIOORGANIC & MEDICINAL CHEMISTRY LETTERS, PERGAMON, AMSTERDAM, NL, vol. 22, no. 1, 8 November 2011 (2011-11-08), pages 225-230, XP028352308, ISSN: 0960-894X, DOI: 10.1016/J.BMCL.2011.11.059 [retrieved on 2011-11-23]
- WANG, Z. ET AL.: 'Identifying Novel Molecular Structures for Advanced Melanoma by Ligant-Based Virtual Screening' JOURNAL OF CHEMICAL INFORMATION AND MODELING vol. 49, 2009, pages 1420 - 1427, XP009170439
- BORCHHARDT, D.M. ET AL.: 'Biochemical Evaluation of a Series of Synthetic Chalcone and Hydrazine Derivates as Novel Inhibitors of Cruzain from Trypanosoma cruzi' JOURNAL OF THE BRAZILIAN CHEMICAL SOCIETY vol. 21, no. 1, 2010, pages 142 - 150, XP055069491
- JIN, L. ET AL.: 'Synthesis, structure, and bioactivity of N'-substituted benzylidene- 3,4,5- trimethoxybenzohydrazide and 3-acetyl-2-substituted phenyl-5-( 3,4,5-trimethoxyphenyl)-2,3-dihydro-1,3,4-o xadiazole derivates' BIOORGANIC & MEDICINAL CHEMISTRY LETTERS vol. 16, 2006, pages 5036 - 5040, XP005611690
- CAO, X. ET AL.: 'Synthesis and Biological Activity of a Series of Novel N-Substituted p-Lactams Derived from Natural Gallic Acid' JOURNAL OF THE CHINESE CHEMICAL SOCIETY vol. 58, 2011, pages 35 - 40, XP055069493
- SALUM LÍVIA B ET AL: "N-(1'-naphthyl)-3,4,5-trimethoxybenzohydr azide as microtubule destabilizer: Synthesis, cytotoxicity, inhibition of cell migration andin vivoactivity against acute lymphoblastic leuke", EUROPEAN JOURNAL OF MEDICINAL CHEMISTRY, vol. 96, 23 February 2015 (2015-02-23), pages 504-518, XP029168199, ISSN: 0223-5234, DOI: 10.1016/J.EJMECH.2015.02.041

## Description

### Field of the Invention

The present invention relates to acyl-hydrazones derived from 3,4,5-trimethoxyphenyl hydrazine for the treatment of diseases associated with cell proliferation (such as leukemias, tumors, inflammation and other proliferative diseases). More specifically, the invention relates to compounds where the action mechanism is through inhibition of tubulin, which may therefore be useful in treating acute lymphoblastic leukemia (ALL). The invention further relates to obtaining novel acyl-hydrazones and oxadiazoles from 3,4,5-trimethoxyphenyl-hydrazine.

### Background of the Invention

Cancer is a disease characterized by proliferation and uncontrolled spread throughout the body of abnormal forms of human cells. The neoplastic cells differ from normal cells by the high invading capability they possess, by the loss of function, by the loss of differentiation and by the ability to metastasize, by virtue of having lower adherence among themselves (Rang, H. P.; Dale, M. M.; Ritter, J. M; Moore, P. K. Farmacologia. 5 ed. Rio de Janeiro: Elsevier, 2004. 703 p). In 2009, it was estimated the occurrence of 49,000 new cases of breast cancer, 47,000 of prostate cancer, 27,000 of lung cancer, 25,000 of colon and rectum cancer, 23,000 of stomach cancer and 19,000 of cervical cancer in Brazil in 2009 (INCA, Instituto Nacional do Câncer [National Cancer Institute]. Available at: <http://www.inca.gov.br/estimativa/2006/index.asp?link=mapa.asp&ID=8> Accessed on: February 23, 2011), being the second leading cause of death in the country.

Leukemia is one of several types of cancer, and occurs by neoplastic proliferation of lymphoid or myeloid hematopoietic cells, resulting from the mutation of a single stem cell, whose progeny form a clone of leukemic cells. Multiple genetic alterations for malignant transformation usually occur, including inappropriate expression of oncogenes and loss of function of tumor suppressor genes (Bain, B. J. Diagnóstico em leucemias. Rio de Janeiro: Elsevier, 2003, Cap. 1, 01-56), that may be associated with genetic factors or risks (such as smoking, exposure to radiation or chemicals such as benzene) (INCA, Instituto Nacional do Câncer [National Cancer Institute] - Leucemia - prevenção, genética, outros fatores de risco [Leukemia - prevention, genetics, other risk factors] available at: <http://www2.inca.gov.br/wps/wcm/connect/tiposdecancer/site/home/leucemia/prevenca o_genetica_outros_fatores_de_risco>. Accessed on: February 23, 2011; and IARC (International Agency for Research on Cancer). World Cancer Report 2008. Available at: <http://www.iarc.fr/en/publications/pdfs-online/wcr/2008/index.php> and < http://www.iarc.fr/en/publications/pdfs-online/wcr/2008/wcr_2008.pdf >. Accessed on: February 23, 2011). Leukemias are further subdivided based on how quickly the disease progresses and becomes severe, and may be acute or chronic. Acute leukemias are characterized by a defect in cell maturation, which leads to an
imbalance between proliferation and maturation; since the leukemic clone of cells continue to proliferate without reaching the stage of maturation and death, leading to a continued expansion of the leukemic clone and predominance of immature cells (INCA 2009b, Bain, B. J. Diagnóstico em leucemias. Rio de Janeiro: Elsevier, 2003, Cap. 1, 01-56).

Acute lymphoblastic leukemia (ALL) is due to the uncontrolled proliferation of immature lymphoid progenitor cells in the bone marrow, resulting in a very rapid accumulation of neoplastic cells (Plasschaert, S.; Van Der Kolk, D.; De Bont, E.; Vellenga, E.; Kamps, W.; De Vries, E. Breast Cancer Resistance Protein (BCRP) in Acute Leukemia. Leukemia & Lymphoma, 2004, 45, 649-654). It accounts for 80% of the cases of acute leukemia in childhood (Laks, D.; Longhi, F., Bernardes, W. M.; Ramos, G. P. C. J Pediatr, 2003, 79, 149-158) and for 50% of all hematopoietic malignancies (Downing, James R.; Shannon, Kevin M. Acute leukemia: A pediatric perspective. Cancer Cell, 2002, 2, 437-445). In adults, ALL is relatively rare, accounting for 2-3% of hematopoietic malignancies (Downing, James R.; Shannon, Kevin M. Acute leukemia: A pediatric perspective. Cancer Cell, 2002, 2, 437-445); however, the prognosis is much worse than for children because it affects multipotent stem cells, yielding a much more aggressive leukemia (Greaves, M. F. Stem cell origins of leukaemia and curability. British Journal of Cancer, 1993, 67, 413-423) .

The research on the use of compounds from natural sources as chemotherapy is ample. An example is the antineoplastic paclitaxel (Taxol ™), one of the most important antitumor natural products ever discovered, first reported in 1971 (Wani, M. C.; Taylor, H. L.; Wall, M. E.; Coggon, P.; McPhail, A. T. Plant antitumor agents. VI. Isolation and structure of taxol, a novel antileukemic and antitumor agent from Taxus brevifolia. Journal of the American Chemical Society, 1971, 93, 2325-2327), that only after a long period has been approved by the FDA for clinical use (in 1992). Another example is vincristine, an alkaloid used in the therapy for acute leukemia and other types of tumors, which operates the same way as paclitaxel by binding to tubulin, interfering with the formation (polymerization) or reorganization (depolymerization) of microtubules (Goodman e Gilman. As bases farmacológicas da terapêutica, 10a Ed., editora Mc Graw Hill. 2006).

The most commonly used chemotherapeutic agents in the current therapy for leukemia include daunorubicin, doxorubicin, dexamethasone, vincristine, methotrexate and mercaptopurine (Plasschaert, S.; Van Der Kolk, D.; De Bont, E.; Vellenga, E.; Kamps, W.; De Vries, E. Breast Cancer Resistance Protein (BCRP) in Acute Leukemia. Leukemia & Lymphoma, 2004, 45, 649-654). These drugs provide therapeutic benefit, but also significant toxicity to the body and normal cells, due to their role in the induction of apoptosis and inhibition of cell proliferation (Herr, I.; Debatin, K. M. Cellular stress response and apoptosis in cancer therapy. Blood 2001, 98, 2603-2614; and Leszczyniecka, M.; Roberts, T.; Dent, P.; Grant, S.; Fisher, P. B. Differentiation therapy of human cancer: basic science and clinical applications. Pharmacology & Therapeutics, 2001, 90, 105-156). Antineoplastic agents also interfere with normal tissues that have rapidly dividing cells, which can cause many undesirable effects, such as reducing the production of defense cells in the body, poor wound healing, alopecia, gastrointestinal epithelial injury, sterility and teratogenicity (Rang, H. P.; Dale, M. M.; Ritter, J. M; Moore, P. K. Farmacologia. 5 ed. Rio de Janeiro: Elsevier, 2004. 703 p).

Despite recent advances in cancer research, in 2008 there were 5,686 registered deaths from leukemia in Brazil, and for 2010 there were an estimated 9,580 new cases of the disease (INCA, Instituto Nacional do Cancer [National Cancer Institute]. Available at: < http://www.inca.gov.br/estimativa/2006/index.asp?link=mapa.asp&ID=8> Accessed on: February 23, 2011), which constantly motivates the search for new drugs for the treatment of leukemia and other tumors.

In a recent study, Wang and colleagues (Wang, Z.; Lu, Y.; Seibel, W.; Miller, D. D.; Li, W. Identifying Novel Molecular Structures for Advanced Melanoma by Ligand-Based Virtual Screening. Journal of Chemical Information and Modeling, 2009, 49, 1420-1427) evaluated new antitumoral compounds based on the structure of the compound LY-1-100, which has a proven mechanism of action and affinity for microtubules (colchicine binding site), but low selectivity.

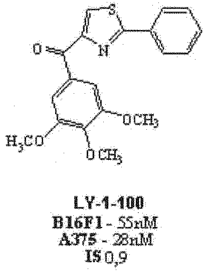

Structures were selected by ligand-based virtual screening, resulting in 14 new molecules with high structural similarity to the starting compound (LY-1-100). The theoretical results obtained by researchers revealed that the trimethoxylated ring of the LY-1-100 compound must be maintained to provide antitumor activity, but that the triazole ring doesn't seem to be important and could be replaced by the structure of *N*-methylene-hydrazine, which generates the structure of acyl-hydrazones. Although the results in murine B16F1 and A375 cells (in vitro) of these compounds have not shown improved potency and selectivity on the starting compound, these results were very promising and identified some major structural components for anti-melanoma activity (Wang, Z.; Lu, Y.; Seibel, W.; Miller, D. D.; Li, W. Identifying Novel Molecular Structures for Advanced Melanoma by Ligand-Based Virtual Screening. Journal of Chemical Information and Modeling, 2009, 49, 1420-1427).

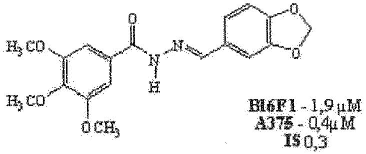

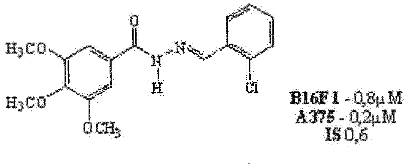

In this regard, acyl-hydrazones appear as an interesting class of compounds with antitumor activity. Thus, the present invention relates to obtaining acyl-hydrazones, especially those derived from 3,4,5-trimethoxyphenyl-hydrazide for the treatment of diseases associated with cell proliferation (such as leukemias, tumors, inflammation and other proliferative diseases).

The patent application PI 0112674-1 (n-[5-[[[5-alkyl-2-oxazolyl]methyl]thio]-2-tiazolyl]carboxamide inhibitors of cyclin-dependent kinases) describes compounds and their enantiomorphs, diastereomers, solvates and pharmaceutically acceptable salts thereof as inhibitors of protein kinases useful in the treatment of proliferative diseases such as cancer, inflammation and arthritis. The synthesized compounds can also be useful for the treatment of Alzheimer's disease, chemotherapy-induced alopecia and cardiovascular disease. The compounds described in the application PI 0112674-1 represent more complex structures than those presented in the course of the present invention.

The compounds of the referenced patent application publication No. U.S. 20040138272 (1,4-Substituted cyclohexane derivatives) may be useful in preventing cell proliferation in malignant diseases by inhibiting Rho kinases, useful in the repair of central and peripheral nervous system by growth induction and regeneration of axons. The mechanism of action of the compounds of publication U.S. 20040138272 differs from that proposed for the structures of the present invention.

Inhibitors of cyclin-dependent kinases (cdks) useful in modulating cell-cycle progression are proposed in patent application PI 0418095-0 (inhibitor of cyclin-dependent kinases, compositions and uses related thereto). Such compounds would be useful for the treatment of patients with disorders associated with excessive cell proliferation. The compounds described in PI 0418095-0 are acyl-hydrazones different from those proposed in the course of the present invention with a more complex synthesis process.

In the patent application PI 0508364-8 (derivatives of 4-benzimidazol-2-yl-pyridazin-3-one) compounds are described and physiologically tolerated salts thereof, which have activity as inhibitors of kinases, in particular CDK2 kinase (cyclin-dependent kinase 2). The compounds of PI 0508364-8 are different from the proposed in the course of the present invention with a more complex synthesis.

The publication of U.S. patent application No. U.S. 20070066610 (Acyl-hydrazones as kinase modulators) discloses acyl-hydrazones as inhibitors of tyrosine kinases, comprising c-Met, a tyrosine kinase receptor which regulates cellular proliferation, morphogenesis, and motility. The acyl-hydrazones described in U.S. 20070066610 are different from those proposed in the course of this invention, with a more complex synthesis. In addition, the target of action of the compounds described differs from the proposed herein.

The published U.S. patent application 20080194562 (Pyrazole Derivatives for The Inhibition of Cdk's And Gsk's) refers to the synthesis of pyrazole compounds that inhibit or modulate the activity of cyclin-dependent kinases (CDK) and kinase glycogen synthase (GSK), and their use in the treatment or prophylaxis of kinase mediated diseases or conditions. Pharmaceutical compositions containing the compounds and chemical intermediates are also described. The compounds of the U.S. 20080194562 document are different from the proposed in the course of the present invention.

Acyl-hydrazones are also described in the literature for their pronounced insecticidal and plant growth stimulation activities (Robinson, B. Fischer indole synthesis. Chem. Rev., 1963, 4, 373-401); for the treatment of tuberculosis (Vigorita, M. G.; Ottana, R.; Zappala, C.; Maccari, R.; Pizzimenti, F. C.; Gabbrielli, G. Halogenated isoniazid derivatives as possible antimycobacterial and anti-HIV agents - III. Farmaco, 1994, 49, 775-781); and as bacteriological and bacteriostatic agents (Samus, N. M.; Tsapkov, V. I.; Kuracheva, S. A.; Burdenko, T. A. Synthesis and antimicrobial activity of coordination compounds of 3d-elements with some hydrazones derived by using 5-nitro-2-furaldehyde. Khimiko-Farmatsevticheskii Zhurnal, 1994, 28, 41-44).

Considering what has been stated above, a study was conducted with acyl-hydrazones and their oxadiazole derivatives obtained by the non-classic bioisosterism strategy of ring closure, with the goal of developing new chemotherapeutic agents. The oxadiazoles are an important class of heterocyclic compounds with a wide range of biological activities, such as antiviral, antimicrobial, antineoplastic, fungicidal, and inhibition of tyrosinase and cathepsin K (Kumar, D.; Sundaree, S.; Johnson, E. O.; Shah, K. An efficient synthesis and biological study of novel indolyl-1,3,4- oxadiazoles as potent anticancer agents. Bioorganic & Medicinal Chemistry Letters, 2009, 19, 4492-4494). Also, they are great bioisosters of amides and esters, which can substantially contribute to increased pharmacological activity, participating in hydrogen bonds as receptors (Guimaraes, C. R. W.; Boger, D. L.; Jorgensen, W. L. Elucidation of Fatty Acid Amide Hydrolase Inhibition by Potent α-Ketoheterocycle Derivatives from Monte Carlo Simulations. Journal of the American Chemical Society, 2005, 127, 17377-17384).

Thus, the specific acyl-hydrazones and their analogues, from the processes from this invention described in detail below, as well as their use in the treatment of leukemia, tumors and other proliferative diseases such as inflammation, is of great economic and social interest.

US2011/1 89306 describes therapeutic and/or prophylactic uses of hydrazide compounds and pharmaceutical compositions containing one of more of these compounds as active component for treating a disease or disorder requiring modulation of vacuolar (H+)-ATPases.

CN1824657 describes a two-step synthesis process to obtain a 3-substituted-2-aryl substituted -5-(3,4,5-trialkoxyphenyl)-1,3,4-oxdiazole a derivative using 3,4,5-trialkoxybenzoyl hydrazine, aromatic aldehyde and acid anhydride as raw material and methyl alcohol, ethyl alcohol, isopropanol, absolute alcohole, benzene, toluene, xylene, N,N-dimethylformamide, dioxane, methyl-sulfoxide, cyclohexane, n-hexane or their mixture as solvent, and the use of such derivatives for resisting tumor and for inhibiting prostate cancer.

### Goals of the Invention

The aim of the present invention is to obtain synthetic compounds derived from 3,4,5-trimethoxyphenyl-hydrazide (hydrazones and oxadiazoles) and all analogs, and the like, by means of a simple synthetic route, as well as the use of these compounds for the treatment of diseases associated with cell proliferation (such as leukemias, especially acute lymphoblastic leukemia - ALL - tumors, inflammation and other proliferative diseases). The present invention also describes procedures used to determine the biological activity of these compounds.

### Summary of the Invention

The present invention provide compounds according to Claims 1 and 3, and compounds for use according to Claims 2, 5, 6 and 7. The present invention also describes procedures used for the determination of biological activity of all these compounds.

Acyl-hydrazones with a similar activity to the compound used as a standard in the experiments (colchicine) were obtained. The higher selectivity of the compounds disclosed herein is an important feature related with fewer side effects than the drugs currently used in the clinic practice. The acyl-hydrazones synthesized, more specifically the compounds **02** and **07** showed significant antileukemic activity, which indicates **02** and **07** as candidates for drugs prototypes or drugs, for the treatment of leukemias, especially acute lymphoblastic leukemia (ALL), tumors and other proliferative diseases such as inflammation.

The determination of the action mechanism of the most active compounds was performed by using DNA microarrays and subsequent tests indicated through the chip, in addition to selectivity studies in healthy human lymphocytes.

### Description of the Figures

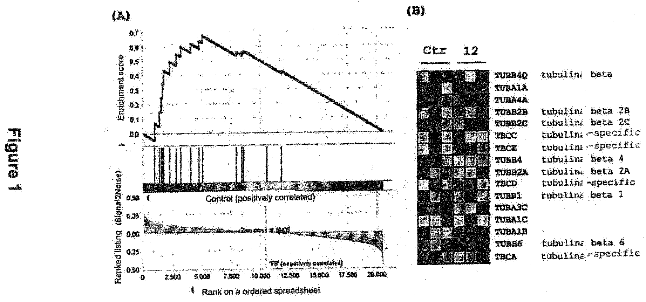

Figure 1 shows (A) Overview of the enrichment of the gene set defined as "REACTOME POST CHAPERONIN TUBULIN FOLDING PATHWAY" showing enrichment in control samples HL-60 versus samples treated with compound 07 (p = 0.002, FDR q-value = 0.168) and (B) The expression values of the 16 genes that are part of the set of genes in (A) are represented as blue squares (lower expression) and red squares (higher expression).

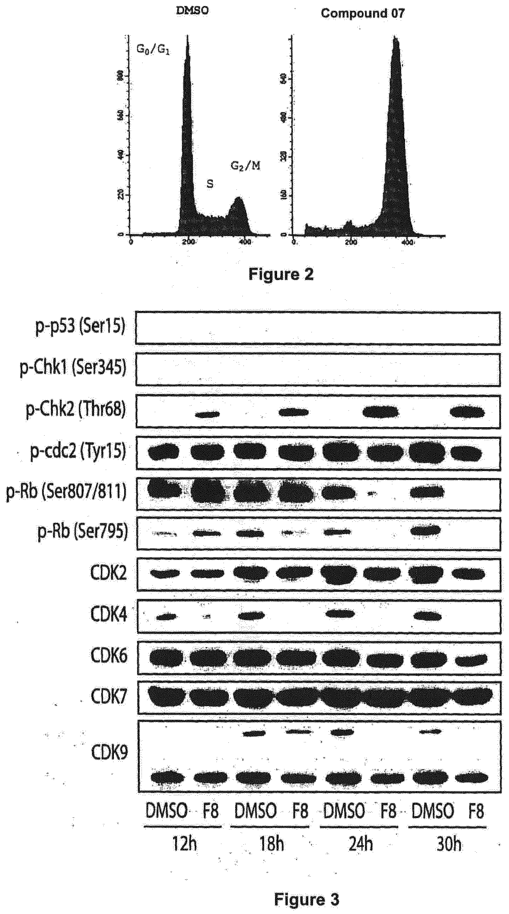

Figure 2 shows that Compound 07 induces cell cycle arrest in G2/M. Jurkat cells treated for 18 h with 125 nM of compound 07 or DMSO were subjected to cell cycle analysis after staining with propidium iodide and flow cytometry analysis.
Figure 3 shows the results of Western blot analysis for various cell cycle regulators protein extracts from Jurkat cells treated with 125 nM of compound 07 (F8) or vehicle (DMSO).

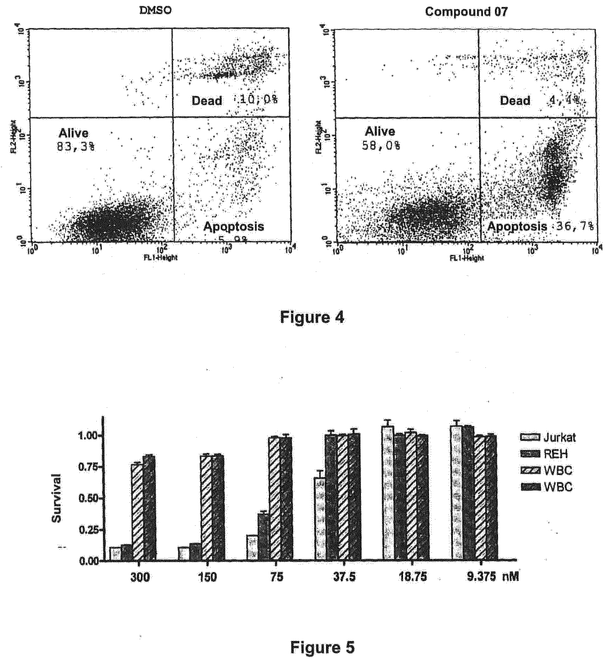

Figure 4 shows that compound **07** is a strong inducer of apoptosis in Jurkat cells. Overall, the results suggest that compound **07** promotes cell cycle arrest and apoptosis, mainly through Chk2 and Rb. Jurkat cells treated for 18h with 125 nM of compound 07 or DMSO were double stained with annexin V *I* propidium iodide and analyzed by flow cytometry.
Figure 5 shows the effect of compound 07 on human lymphocytes (WBC) and leukemic Jurkat and HEK cells after 48h. The proliferation of normal lymphocytes were stimulated with phytohemagglutinin. The percentage of the survival of cells treated with compound 07 versus the survival of cells treated with vehicle (DMSO) is shown.

### Detailed Description of the Invention

The present invention comprises obtaining and the action mechanism of synthetic acyl-hydrazones and its analog and related compounds, which may be useful in treating leukemias, especially acute lymphoblastic leukemia (ALL), tumors and other proliferative disorders such as inflammation.

According to one of the aspects of the present invention, the present invention provides a compound of formula (I) 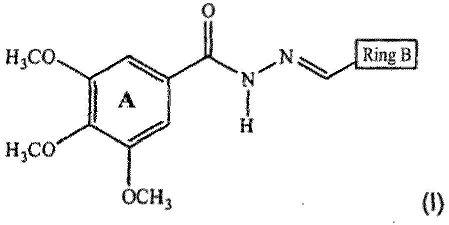 wherein Ring B is a substituent selected from the group comprising 3-OCH₂CH₃-4-OH-phenyl; and 2-OH-4-Br-phenyl;.

The compounds of formula I described above and compounds of formula I wherein Ring B is a substituent selected from

| | |
|---|---|
| 01 - phenyl | |
| 02 - 4-Br-phenyl | 24 - 3-OCH₂CH₃-4-OH-phenyl |
| 03 - 4-NO₂-phenyl | |
| 07 - 1-naphthyl | 25 - 3-OCH₃-phenyl |
| 08 - 2 naphthyl | 26 - 2-OH-4-Br-phenyl |
| 09 - 4-Cl-phenyl | |

can be used in treating cancer.

Also described are compounds of formula I wherein Ring B is a substituent selected from:
2-naphthyl; 3,4-OCH₂O-phenyl; 2-Cl-phenyl; 2,5-diOCH₃phenyl; 3,4,5-triOCH₃-phenyl; and 4-CF₃-phenyl.

Also described herein is a further group of compounds having the structure II: 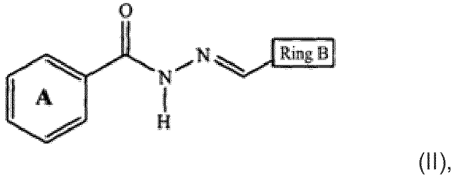 wherein Ring B represents:
30 - phenyl
31 - 3,4-OCH₂O-phenyl
32 - 4-Br-phenyl
33 - 4-CH₃-phenyl
34 - 1-naphthyl
35 - 2-naphthyl

Additionally, synthetic analogs oxadiazoles of the present invention have the structure III: 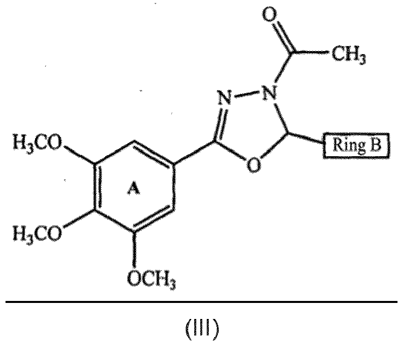 wherein ring B represents:
36-3-OCH₃-4-OH-5-Br-phenyl
37 - 3-OCH₃-4-OH-phenyl 38 - 1-naphthyl

The present invention also provides a compound of formula Ia or Ila 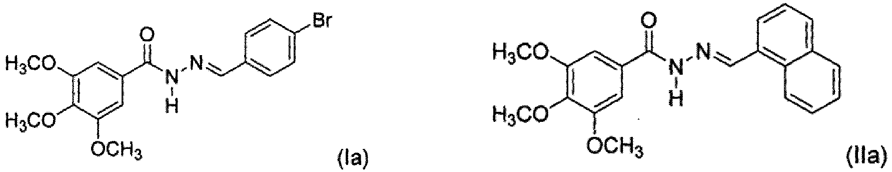 for use in the treatment of a disease associated with cell proliferation, wherein the disease associated with cell proliferation is selected from acute lymphoblastic leukemia (ALL), tumours and inflammation.

Acyl-hydrazones including those of the present invention were obtained from the condensation reaction between 3,4,5-trimethoxyphenyl-hydrazide and different aldehydes using ethanol as solvent at reflux, according to the reaction: 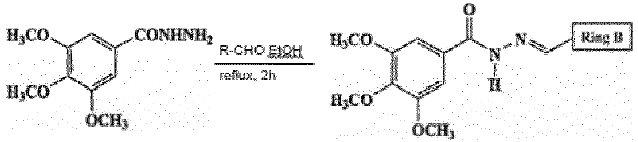 wherein ring B represents:
02 - 4-Br-phenyl*
05 - 3-OCH₃-4-OH-5-I-phenyl
07 - 1-naphthyl*
17 - 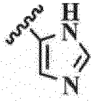
21 - 2,6-diOCH₃-phenyl
26 - 2-OH-4-Br-phenyl
29 - 3-CF₃-4-Cl-phenyl

Table 1 shows the yields obtained in the synthesis and the experimental melting points of the 3,4,5-trimethoxyphenyl-hydrazones.

**Table 1. Yields and melting points of synthesized 3,4,5 trimethoxyphenyl-hydrazones.**

| | | | |
|---|---|---|---|
| 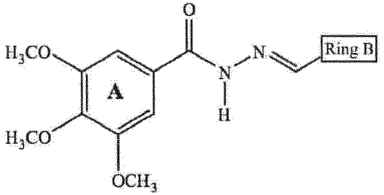 | | | |

| **Compound nr.** | **Ring B** | **Yield (%)** | **Experimental melting point (°C)** |
|---|---|---|---|
| **02*** | 4-Br-phenyl | 82 | 208-209 |
| **05** | 3-OCH₃-4-OH-5-I-phenyl | 69 | 247-248 |
| **07*** | 1-naphtyl | 82 | 229-230 |
| **17** | 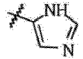 | 75 | 230-231 |
| **21** | 2,6-diOCH₃-phenyl | 93 | 245-246 |
| **26** | 2-OH-4-Br-phenyl | 65 | 206-208 |
| **29** | 3-CF₃-4-Cl-phenyl | 83 | 203-204 |

The structure of compound **02** was previously published as the reaction intermediate for obtaining oxadiazole (Mazzone, G.; Bonina, F.; Formica, F. Some aroylhydrazones of halobenzaldehydes and halo-substituted 2,5-diaryl-1,3,4-oxadiazoles. Farmaco, Edizione Scientifica, 1978, 33(12), 963-71) and compound 07 was previously evaluated as a MAO (monoamine oxidase) inhibitor (Mazzone, G.; Arrigo Reina, R. 3,4,5-Trimethoxybenzoyl hydrazides and their anti-MAO [monoamine oxidase] activity. Bollettino delle Sedute della Accademia Gioenia di Scienze Naturali in Catania, 1971, 10(8), 689-702). The chemical characterization of both compounds (**02** and **07**) was also previously published by our research group, in a study that evaluated the activity of these and other compounds as inhibitors of *Trypanosoma cruzi* cruzain, however, the compounds **02** and **07** did not show inhibitory activity of this protein (Borchhardt, Deise M.; Mascarello, Alessandra; Chiaradia, Louise Domeneghini; Nunes, Ricardo J.; Oliva, Glaucius; Yunes, Rosendo A.; Andricopulo, Adriano D. Biochemical evaluation of a series of synthetic chalcone and hydrazide derivatives as novel inhibitors of cruzain from Trypanosoma cruzi. Journal of the Brazilian Chemical Society, 2010, 21(1), 142-150). However, the use of the compounds 02 and 07 for the treatment of leukemia is not described in the prior art and is within the scope of the invention.

The additional acyl-hydrazones (prepared to assist in the discussion of biological tests) were synthesized by the reaction between the hydrazide and phenyl different aldehydes by the same procedure for the preparation of hydrazides described above, according to the following reaction: 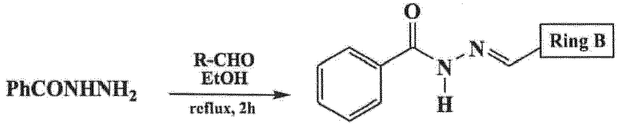 wherein Ring B represents:
30 - phenyl
31 - 3,4-OCH₂O-phenyl
32 - 4-Br-phenyl
33 - 4-CH₃-phenyl
34 - 1-naphthyl
35 - 2-naphthyl

Novel synthetic analogs oxadiazoles of 3,4,5-trimethoxyphenyl-hydrazones were also prepared, from the reaction of these with acetic anhydride according to the following reaction: 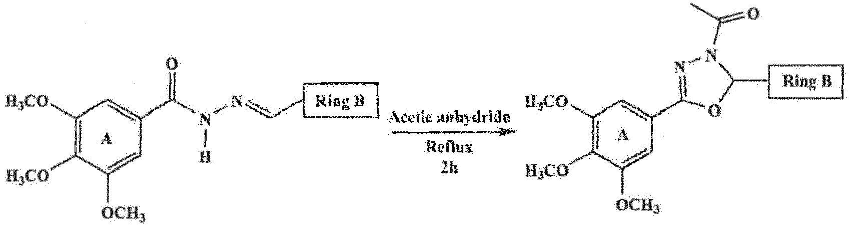 wherein ring B represents:
36 - 3-OCH₃-4-OH-5-Br-phenyl
37 - 3-OCH₃-4-OH-phenyl
38 - 1-naphthyl

Table 2 shows the yields obtained in the synthesis and experimental melting point of the oxadiazoles.

**Table 2. Yields and melting point of the novel synthesized oxadiazoles.**

| | | | |
|---|---|---|---|
| 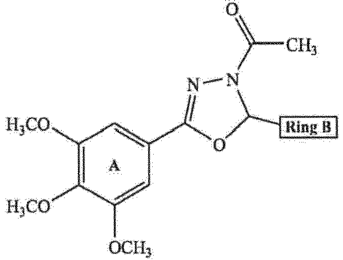 | | | |

| **Compound nr.** | **Ring B** | **Yield (%)** | **Experimental melting point (°C)** |
|---|---|---|---|
| **36** | 3-OCH₃-4-OH-5-Br-phenyl | 30 | 177-179 |
| **37** | 3-OCH₃-4-OH-phenyl | 25 | 170-172 |
| **38** | 1-naphtyl | 35 | 189-192 |

The present invention also describes the determination of the action mechanism of the synthesized acyl-hydrazones and their analogue and similar compounds comprising oxadiazole. The invention also refers to the use of all such compounds as prototype drugs, or drugs, for the treatment of leukemias, especially acute lymphoblastic leukemia (ALL), tumors and other diseases associated with cell proliferation such as inflammation.

The acyl-hydrazones described herein act selectively on leukemic cells with activity in nanomolar order when compared to its activity in healthy human lymphocytes, as will be described below in the form of an example. Shown here is the relevance of biological results, novel for the tested compounds.

In another embodiment, the present invention provides pharmaceutical compositions comprising the compounds described above in combination with excipients, carriers and pharmaceutically acceptable adjuvants.

As used herein, the use of the term "pharmaceutically acceptable" means a non-toxic, inert solid, liquid, semisolid excipient, diluent, auxiliary formulation of any type, or simply a sterile aqueous medium such as saline. Some examples of materials which can serve as pharmaceutically acceptable carriers are sugars such as lactose, glucose and sucrose, starches such as corn starch and potato starch, cellulose and its derivatives such as sodium carboxymethyl cellulose, ethyl cellulose and acetate cellulose, cyclodextrin; oils such as peanut oil, cottonseed oil, sunflower oil, sesame oil, olive oil, corn oil and soybean oil; glycols such as propylene glycol, polyols such as glycerin glycol, sorbitol, mannitol and polyethylene; esters such as ethyl laurate, ethyl oleate, agar; buffering agents such as aluminum hydroxide and magnesium hydroxide; alginic acid; pyrogen-free water; isotonic saline, Ringer's solution; ethyl alcohol and phosphate buffer solutions, as well as other non-toxic compatible substances used in pharmaceutical formulations.

Particularly, the pharmaceutical or veterinary compositions comprising the compounds of the present invention can be intended for administration by any type of administration, especially parenteral administration.

Specifically, the compositions of the present invention may comprise any type of excipient used in the production of drugs in any of the above pharmaceutical forms, such as absorbents, diluents, binders, disintegrants, lubricants, glidants, plasticizers, coating agents, matrix forming agents for controlled release, solvents and co-solvents, wetting agents, emulsifiers, surfactants, thickening agents, tonicity agents, humectants, levigating agents, agents to expel air, alkalizing or acidifying agents, preservatives, antioxidants, bactericides, bacteriostats, chelating agents, colorants and sweeteners.

Absorbents suitable for the compositions of the present invention may be any substance added to absorb the water present in the extracts or for setting certain volatile compounds, such as essences. Non-limiting examples of such excipients are calcium phosphate, kaolin, magnesium carbonate bentonite, and talc.

The compositions of the present invention may comprise, as solvent, any inert material added to formula to allow to obtain tablets or filling capsules with appropriate volumes and provide flow and compression properties necessary for production, for example, but not limited to, lactose, tribasic calcium phosphate, starch, mannitol, calcium sulfate, microcrystalline cellulose (Microcel, Avicel), dibasic calcium phosphate (Encompress, Ditab), magnesium oxide, magnesium carbonate, talc, kaolin, calcium carbonate, dextrose fructose, lactose, aspartame, cellulose, maltose, mannitol, guar gum, sorbitol, starch and sucrose.

Suitable binder substances for the compositions of this invention may be agents used to promote adhesion of the particles during granulation and compression of solid pharmaceutical forms, and can also be used in the compositions of the present invention, for example, carbopol, povidone, xanthan gum, gum arabic, alginic acid, compressible sugar, CMC-Na, ethylcellulose, gelatin, methylcellulose, povidone (PVP), starch, pregelatinized starch and liquid glucose in solution, dispersion or powder.

Disaggregants or disintegrants suitable for compositions of the present invention may be any component used for accelerating disintegration and/or dissolution of the pharmaceutical form in biological fluids, for example, alginic acid, starch, sodium alginate, CMC-Na, microcrystalline cellulose, croscarmellose sodium (Ac-Di-sol), sodium starch glycolate (Explotab) and crospovidone (Kollidon CL).

Lubricants suitable for compositions of this invention may be, for example, magnesium stearate, calcium stearate, stearic acid, talc and hydrogenated vegetable oil (e.g. Lubritab).

Glidants suitable for the compositions of the present invention may be, for example, colloidal silica (Aerosil 200) and talc.

Plasticizer agents suitable for the compositions of the present invention can be used with polymers to modify the phase transition temperature thereof and facilitate coalescence of formed films on granules, tablets or pellets. Non-limiting examples of such agents are glycerin, triethyl citrate, dibutyl phthalate, silicone, and PPG.

Coating agents used for coating compositions of the present invention in the form of tablets, granules, capsules or pellets may be, for example, cellulose acetate phthalate, ethylcellulose, gellan gum, maltodextrin, methacrylates, methylcellulose, microcrystalline cellulose, shellac, carrageenan gum, waxes, shellacs, gelatin, cellulose derivatives (methyl or ethyl cellulose, cellulose acetate phthalate, hydroxypropyl methyl cellulose, cellulose acetate), copolymers of acrylic and methacrylic esters (Eudragit of type L100, RS 30D, RS PM, S100, among others), polyvinyl alcohol (PVA) and polyvinyl acetate.

Matrix forming agents for the controlled release possibly employed in the compositions of the present invention, in order to obtain extended and/or controlled release of the active principle(s) may be HPMC, CMC-Na, xanthan gum, Carbopol, various types of Eudragit, agar-agar, polyoxyethylene derivatives (PEO's), cyclodextrin nanospheres and nanoparticles of any nature.

Solvents and co-solvents such as ethanol, corn oil, cottonseed oil, glycerin, isopropyl alcohol, mineral oil, oleic acid, peanut oil, purified water, water for injection, among others, can also be used in the compositions of the present invention.

Wetting agents suitable for the compositions of the present invention may be any substance added for the purpose of reducing the surface tension at the liquid/solid interface, for example, sodium lauryl sulfate (SLS), sodium docusate and polysorbates 20, 60, 80 (Tween 20, 60, 80).

Emulsifying agents suitable for the compositions of the present invention may be, for example, glyceryl monostearate, cetyl alcohol and gelatin and auxiliaries such as CMC-Na, MC, alginate and pectin.

Surfactant agents such as, for example, benzalkonium chloride, nonoxinol 10, octoxinol 9, polysorbate 80 and sodium lauryl sulfate are also adequate for the compositions of the present invention.

Thickening agents suitable for the compositions of the present invention may be any substance used to increase the consistency of ointments, for example, cetyl alcohol, white wax, yellow wax, stearyl alcohol, paraffin, microcrystalline wax, and cetyl esters wax.

Tonicity agents suitable for the compositions of the present invention may be any substance used to obtain solutions having osmotic characteristics similar to those of biological fluids to be administered by the ocular, nasal, parenteral routes such as NaCl (0.9%), mannitol (5.07%) and dextrose (5.51 %).

Humectants suitable for compositions of the present invention can be glycerin, propylene glycol and sorbitol.

Levigating agents suitable for the compositions of this invention can be any liquid used as a facilitating agent in the process of reducing the drug particles during preparation of emulsions, oily bases, among others, for example, mineral oil (liquid petrolatum), glycerin, propylene glycol process, PEG 400, cottonseed oil, castor oil and polysorbate 80 (Tween ® 80).

Agents for expelling air suitable to the compositions of this invention may be employed to expel the air from hermetically sealed enclosures or from fluid formulations to increase the stability, for example nitrogen (N₂) and carbon dioxide (CO₂).

Alkalizing or acidifying agents such as citric acid, ammonia, acetic acid, ammonium carbonate, fumaric acid, diethanolamine, hydrochloric acid (HCl), monoethanolamine, tartaric acid, potassium hydroxide (KOH), boric acid, sodium hydroxide (NaOH), sodium bicarbonate, sodium borate, and triethanolamine are also suitable for the compositions of the present invention.

Preservatives that can be used in the compositions of the present invention are, for example, antifungal agents such as benzoic acid, sodium benzoate, sodium butylparaben, methylparaben (Nipagin), propylparaben (Nipasol), ethylparaben, sodium propionate, and antibacterials such as benzalkonium chloride , benzethonium chloride, benzyl alcohol, cetylpyridinium, chlorobutanol and phenol chloride.

Antioxidants suitable for the compositions of the present invention may be selected from the group comprising butylated hydroxyanisole (BHA), butylated hydroxytoluene (BHT), β-tocopherol, ascorbic acid, ascorbyl palmitate, sodium metabisulfite, ethylenediamine tetraacetic acid (EDTA), citric acid, cysteine, glutathione vitamin C, sodium metabisulfite, cysteine and sodium thiosulfate.

The compositions of the present invention may further comprise, as buffering agents, citrate buffer, phosphate buffer and borate buffer. As colorants, flavors and flavorings, vanilla, menthol, cinnamon oil, anise oil and cocoa can be used, for example. Sweeteners may include, for example, aspartame, dextrose (glucose), mannitol, sorbitol, saccharin, sodium cyclamate, sugar, acesulfame potassium, stevioside and sucralose.

The compositions of the present invention may further comprise excipients such as bactericides, bacteriostats, antioxidants, preservatives, buffers, stabilizers, pH adjusters, osmolarity adjusters, antifoaming agents and surfactants, as well as residue of inactivation agents or fractionation antigens, components of growth media and solvents commonly used in the production of medicines. Examples of these types of components can be found in "The Handbook of Pharmaceutical Excipients" (RAYMOND C. Rowe, Publisher The Pharmaceutical Press).

A variety of routes of administration of the compositions described herein is available. The particular mode selected will depend on the particular active ingredient selected, the dosage required for therapeutic efficacy and the patient to which the composition is administered.

In another embodiment, the present invention describes the use of the compounds and compositions described herein for the treatment of diseases associated with cell proliferation, such as acute lymphoblastic leukemia (ALL), tumors and inflammation.

In another embodiment, the present invention provides methods of treating diseases associated with cell proliferation such as acute lymphoblastic leukemia (ALL), tumors and inflammation using the compounds and compositions described herein for the treatment of diseases associated with cell proliferation such as acute lymphoblastic leukemia (ALL), tumors and inflammation.

### Examples

The present invention will now be described by way of illustrative examples.

### Example 1. General procedure for the preparation of 3,4,5-trimethoxyphenyl-hydrazones 01-29

For the synthesis of 3,4,5-trimetoxiphenyl-hidrazones **01-29,** we used the methodology described by Troeberg and col. (Troeberg, L.; Chen, X.; Flaherty, T. M.; Morty, R. E.; Cheng, M.; Hua, H.; Springer, C.; McKerrow, J. H.; Kenyon, G. L.; Lonsdale-Eccles, J. D.; Coetzer, T. H. T.; Cohen, F. E. Chalcone, acyl hydrazide, and related amides kill cultured Trypanosoma brucei brucei. Molecular Medicine, 2000, 6, 660-669). In a 100 mL / 1 mouth reaction flask the 3,4,5-trimethoxyphenyl-hydrazide is placed (2 mmol), prepared as described in Example 2, in an organic solvent: acetone, ethyl acetate, ethyl ether, ethanol, methanol (20mL) and the appropriate aldehyde (2 mmol). The mixture was refluxed for 1 to 10 hours. Then, the solution was filtered and the solid recrystallized in an organic solvent.

### Example 2. General Procedure for obtaining 3,4,5-trimethoxyphenyl-hydrazide used to obtain 3,4,5-trimethoxyphenyl-hydrazones 01-29

For the synthesis of 3,4,5-trimethoxyphenyl-hydrazide used in the preparation of acyl-hydrazones **01-29,** we used the methodology already described (Chida, A. S.; Vani, P. V. S. N.; Chandrasekharam, M.; Srinivasan, R.; Singh, A. K. Synthesis of 2,3-dimethoxy-5-methyl-1,4-benzoquinone: a key fragment in coenzyme-Q series. Synthetic communications, 31, 657-660, 2001), carried out in two steps:
*Obtaining ester:* In a 1000 mL / 1 mouth reaction flask, gallic acid (50g, 0.294 mol) was placed with dimethyl sulfate (178.1 g, 1.413mol), anhydrous potassium carbonate (175.5 g, 1.293mol) and TBAI (tetrabutylammonium iodide) (1g) in an organic solvent which may be ethanol, ethyl ether, ethyl acetate, acetone, petroleum ether (375ml) and refluxed for 1 to 12 hours. The solid obtained was filtered and washed with the same organic solvent (3 x 50 mL). The ester was obtained in the form of a cream-colored amorphous solid, with a 78% yield; mp: 84°C (lit. mp 82-83°C). NMR ¹H (CDCl₃): 1.60 (s, 3H, CH₃), 3.92 (s, 9H, OCH₃), 7.33 (s, 2H, Ar).
*Obtaining hydrazide:* In a 1000 mL / 1 mouth reaction flask, the ester obtained in the first step was placed (48g, 0.212 mol), with a solution of 99% hydrazine hydrate (N₂H₄.H₂O) (77.6g, 1.54mol) and an organic solvent which may be ethanol, ethyl acetate, dichloromethane, acetone, methanol (200 mL). The mixture was refluxed for 1 to 5h and maintained overnight under magnetic stirring only at a temperature between 0 and 50°C. The solid obtained was filtered and recrystallized in methanol to obtain the 3,4,5-trimethoxyphenyl-hydrazide as white crystals with a yield of 85%; mp: 162-163°C (lit. mp 168°C). NMR ¹H (CDCl₃): 3.80 (s, 3H, OCH₃), 3.90 (s, 6H, OCH₃), 7.18 (s, 2H, Ar), 9.55 (NH).

### Example 3. General procedure for the preparation of phenyl-hydrazones 30-35

For the synthesis of phenyl-hydrazones 30-35, we used the methodology described by Troeberg and col. (Troeberg, L.; Chen, X.; Flaherty, T. M.; Morty, R. E.; Cheng, M.; Hua, H.; Springer, C.; McKerrow, J. H.; Kenyon, G. L.; Lonsdale-Eccles, J. D.; Coetzer, T. H. T.; Cohen, F. E. Chalcone, acyl hydrazide, and related amides kill cultured Trypanosoma brucei brucei. Molecular Medicine, 2000, 6, 660-669), in the same way as described for obtaining the 3,4,5-trimetoxiphenyl-hidrazones.

### Example 4. General procedure for the preparation of oxadiazoles 36-38

For the synthesis of oxadiazoles 36-38, we used the methodology described by Jin and col. (Jin, L.; Chen, J.; Song, B.; Chen, Z.; Yang, S.; Li, Q.; Hu, D.; Xu, R. Bioorg Med Chem, 2006, 16, 5036-5040). In a 100 mL / 1 mouth reaction flask, the corresponding 3,4,5-trimetoxiphenyl-hidrazone (1 mmol) and acetic anhydride (10mL) were mixed. The mixture was refluxed for 1 to 10 hours and it was cooled afterwards with the addition of crushed ice and left overnight at a temperature between 0 and 60°C to precipitate the product. The solid obtained was filtered, washed with water and recrystallized with organic solvent/water.

### INFRARED (IR) SPECTRAL DATA AND NUCLEAR MAGNETIC RESONANCE (NMR) OF ¹H AND ¹³C OF THE NOVEL COMPOUNDS

* □□ppm relative to TMS, *Multiplicity* (J in Hz). Solvent CDCl₃.
**05** - ¹H NMR (DMSO-d₆) □ 3.73 (s, 3H, *p*-OCH₃), 3.86 (s, 6H, *m*-OCH₃), 3.89 (s, 3H, *m*-OCH₃), 7.22 (s, 2H, H2, H6), 7.34 (s, 1H, H6'), 7.60 (s, 1H, H2'), 8.31 (s, 1H, HC=N), 10.08 (1H, OH), 11.66 (s, 1H, NH). ¹³C NMR (DMSO-d₆) □ 56.78 (*m*-OCH₃), 60.81 (*p*-OCH₃), 85.17 (C3'), 105.84 (C2, C6), 109.72 (C6'), 128.28 (C1), 129.31 (C1'), 130.75 (C2'), 141.03 (C4), 147.35 (C=N), 147.97 (C5'), 149.00 (C4'), 153.36 (C3, C5), 163.16 (C=O). IR □ₘₐₓ/cm⁻¹ 3382 (N-H), 1636, 1228 (C=O), 1565 (C=N), 1290, 1045 (C-O), 2999, 2839, 1585, 1490, 1334, 1137, 997 (Ar) (KBr). 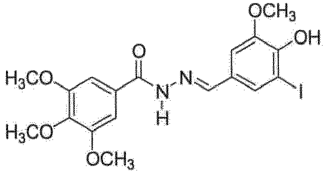
**17** - ¹H NMR (DMSO-de) □ 3.74 (s, 3H, *p*-OCH₃), 3.88 (s, 6H, *m*-OCH₃), 7.23 (s, 2H, H2, H6), 7.53 (s, 1H, H2'), 7.66 (m, 1H, H4'), 8.02 (s, 1H, NH5'), 8.43 (s, 1H, HC=N), 11.37 (s, 1H, NH). ¹³C NMR (DMSO-de) □ 56.76 (*m*-OCH₃), 60.81 (*p*-OCH₃), 105.71 (C2, C6), 129.51 (C1), 131.94 (C1'), 132.16 (C2'), 135.37 (C4'), 145.45 (C=N), 153.35 (C3, C5), 162.29 (C=O). IR □ₘₐₓ/cm⁻¹ 3212 (N-H), 1623, 1234 (C=O), 1580 (C=N), 1280, 1054 (C-O), 2994, 2941, 2838, 1503, 1456, 1411, 1344, 1125, 1006, 844 (Ar) (KBr). 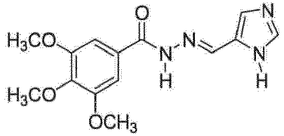
**21** - ¹H NMR (DMSO-d₆) □ 3.71 (s, 3H, *p*-OCH₃), 3.79 (s, 6H, *o*-OCH₃), 3.85 (s, 6H, *m*-OCH₃), 6.72 (d, *J* = 8.0 Hz, 2H, H3', H5'), 7.23 (s, 2H, H2, H6), 7.34 (t, *J* = 8.0 Hz, 1H, H4'), 8.60 (s, 1H, HC=N), 11.52 (s, 1H, NH). ¹³C NMR (DMSO-d₆) □ 56.72 (*m*-OCH₃), 56.78 (*o*-OCH₃), 60.76 (*p*-OCH₃), 105.08 (C3', C5'), 105.76 (C2, C6), 111.75 (C1'), 129.37 (C1), 131.87 (C4'), 140.88 (C4), 143.92 (C=N), 153.31 (C3, C5), 159.38 (C2', C6'), 162.81 (C=O). IR □ₘₐₓ/cm⁻¹ 3186 (N-H), 1644, 1240 (C=O), 1586 (C=N), 1258, 1068 (C-O), 3002, 2928, 2838, 1502, 1473, 1417, 1378, 1342, 1121, 1007, 783 (Ar) (KBr). 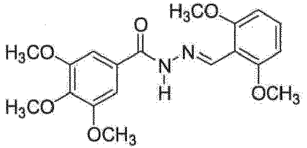
**26** - ¹H NMR (DMSO-d₆) □ 3.42 (s, 3H, *p*-OCH₃), 3.85 (s, 6H, *m*-OCH₃), 6.89 (d, *J* = 8.6 Hz, 1H, H5'), 7.25 (s, 2H, H2, H6), 7.42 (d, *J* = 8.6 Hz, 1H, H6'), 7.78 (s, 1H, H3'), 8.61 (s, 1H, HC=N), 11.26 (s, 1H, NH), 12.01 (1H, OH). IR □ₘₐₓ/cm⁻¹ 3220 (N-H), 1653, 1228 (C=O), 1588 (C=N), 1275, 1099 (C-O), 3004, 2941, 2834, 1550, 1503, 1479, 1463, 1416, 1352, 1335, 1189, 1011, 992, 951, 839, 760 (Ar) (KBr). 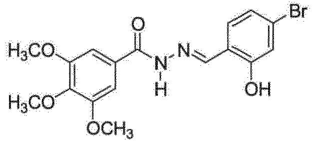
**29** - ¹H NMR (DMSO-de) □ 3.72 (s, 3H, *p*-OCH₃), 3.85 (s, 6H, *m*-OCH₃), 7.22 (s, 2H, H2, H6), 7.82 (d, *J* = 8.6 Hz, 1H, H5'), 8.02 (d, *J* = 8.6 Hz, 1H, H6'), 8.15 (s, 1H, H2'), 8.51 (s, 1H, HC=N), 11.96 (s, 1H, NH). ¹³C NMR (DMSO-d₆) □ 56.65 (*m*-OCH₃), 60.73 (*p*-OCH₃), 105.96 (C2, C6), 126.29 (C2'), 127.98 (C1'), 128.88 (C1), 132.31 (CF₃), 132.91 (C4'), 133.01 (C5'), 134.82 (C6'), 141.27 (C4), 145.61 (C=N), 153.40 (C3, C5), 163.50 (C=O). IR □ₘₐₓ/cm⁻¹ 3182 (N-H), 1655, 1242 (C=O), 1587 (C=N), 1269, 1039 (C-O), 3008, 2938, 2838, 1506, 1480, 1417, 1336, 1316, 1173, 1121, 1006, 958, 666 (Ar) (KBr). 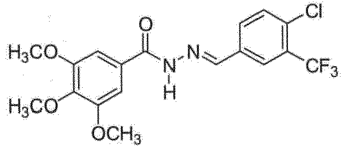
**36** - ¹H NMR (DMSO-d₆) □ 2.36 (s, 3H, CH₃), 3.85 (s, 3H, *p*-OCH₃), 3.91 (s, 3H, *m*-OCH₃), 3.92 (s, 6H, *m*-OCH₃), 6.00 (s, 1H, OH), 6.99 (s, 1H, H2'), 7.06 (s, 1H, H6'), 7.11 (s, 2H, H2, H6), 7.26 (s, 1H, HC-N). ¹³C NMR (DMSO-d₆) □ 21.75 (CH₃), 56.56 (*m*-OCH₃), 61.25 (*p*-OCH₃), 91.52 (C-N), 104.44 (C2, C6), 117.93 (C5'), 119.37 (C2'), 122.52 (C6'), 136.02 (C1', C1), 139.24 (C4'), 141.49 (C4), 152.90 (C3'), 153.64 (C3, C5), 155.84 (C=N), 168.32 (C=O). IR □ₘₐₓ/cm⁻¹ 1766, 1238 (C=O), 1667, 1582 (C=N), 1254, 1047 (C-O), 1177 (C-N), 3445 (OH), 1130, 621 (C-Br), 3004, 2941, 2838, 1466, 1416, 1366, 1306, 1190, 998, 858 (Ar) (KBr). 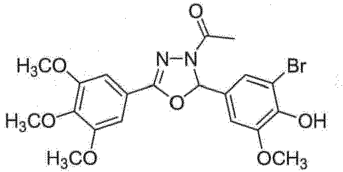
**37** - ¹H NMR (DMSO-d₆) □ 2.35 (s, 3H, CH₃), 3.82 (s, 3H, *p*-OCH₃), 3.90 (s, 3H, *m*-OCH₃), 3.92 (s, 6H, *m*-OCH₃), 6.97 (s, 1H, H2'), 7.06 (m, 1H, H5'), 7.10 (s, 2H, H2, H6), 7.25 (s, 1H, HC-N), 7.44 (m, 1H, H6'); the signal corresponding to the OH group is not observed. ¹³C NMR (DMSO-d₆) □ 21.54 (CH₃), 56.27 (*m*-OCH₃), 56.33 (*m*-OCH₃), 61.02 (*p*-OCH₃), 91.14 (C-N), 104.22 (C2, C6), 109.77 (C2'), 119.16 (C5'), 128.09 (C6'), 136.402 (C1', C1), 141.24 (C4'), 141.96 (C4), 151.78 (C3'), 153.42 (C3, C5), 155.60 (C=N), 168.10 (C=O). IR □ₘₐₓ/cm⁻¹ 1767, 1243 (C=O), 1665, 1581 (C=N), 1250, 1043 (C-O), 1177 (C-N), 3445 (OH), 1129, 644 (C-Br), 2967, 2945, 2838, 1507, 1466, 1417, 1365, 1036, 1287, 1197, 1083, 997, 958, 861, 699 (Ar) (KBr). 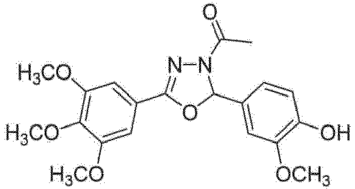
**38** - ¹H NMR (DMSO-d₆) □ 2.47 (s, 3H, CH₃), 3.87 (s, 9H, OCH₃), 7.08 (s, 2H, H2, H6), 7.26 (s, 1H, HC-N), 7.26 (m, 1H, H2'), 7.48 (t, *J* = 8.0 Hz, 1H, H3'), 7.55 (t, *J* = 8.0 Hz, 1H, H7', H8'), 7.62 (t, *J* = 8.0 Hz, 1H, H7'), 7.76 (m, 1H, H4'), 7.92 (d, *J* = 8.0 Hz, 1H, H6'), 8.22 (d, *J* = 8.0 Hz, 1H, H9'). ¹³C NMR (DMSO-d₆) □ 21.57 (CH₃), 56.28 (*m*-OCH₃), 60.98 (*p*-OCH₃), 91.17 (C-N), 104.27 (C2, C6), 119.61 (C1), 123.04 (C2'), 125.07 (C9'), 125.20 (C3'), 126.05 (C7'), 126.95 (C8'), 128.95 (C4'), 130.51 (C6'), 130.61 (C10'), 130.79 (C5'), 134.04 (C1'), 141.11 (C4), 153.29 (C3, C5), 155.83 (C=N), 168.26 (C=O). IR □ₘₐₓ/cm⁻¹ 1731,1243 (C=O), 1669, 1587 (C=N), 1254, 1039 (C-O), 1124 (C-N), 2997, 2941, 2827, 1509, 1465, 1416, 1369, 1332, 1191, 1006, 980, 847, 784, 699 (Ar) (KBr).

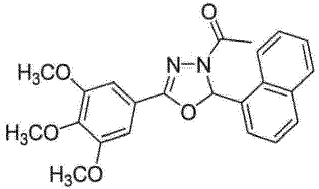

### Example 5. Effect of compounds on acute lymphoblastic leukemia (ALL) cell lineages

a) *Dilution and storage of compounds:* All compounds were resuspended in DMSO at stock concentration of 20 mM and stored at -20°C. For cytotoxicity testing, the dilutions from the stock solutions were made in culture medium (RPMI-1640 plus 10% of fetal bovine serum, 100 IU/ml penicillin and 100 pg/mL Streptomycin), immediately before being discharged to the cells.
b) Assays *of in vitro sensitivity*/*resistance of the cells to the compounds by the MTT method:* The REH and Jurkat cell lines were maintained in RPMI-1640 medium with 10% FBS (fetal bovine serum), 100 IU/mL penicillin, 100 pg/mL streptomycin and incubated at 37°C and 5% CO₂. Cytotoxicity assays were performed according to the method described in the literature (Pieters, R.; et al. In vitro drug sensitivity of cells from children with leukemia using the MTT assay with improved culture conditions. Blood, 1990, 76, 2327-2336; Pieters, R.; et al. Relation of cellular drug resistance to long-term clinical outcome in childhood acute lymphoblastic leukaemia. Lancet, 1991, 338, 399-403 e Kaspers, G. J.; et al. In vitro cellular drug resistance and prognosis in newly diagnosed childhood acute lymphoblastic leukemia. Blood, 1997, 90, 2723). Cells were resuspended at a concentration of 3.75 10⁵ cells/mL in culture medium as described. Eighty microliters of this suspension were seeded into plates with 96 round-bottom wells, containing 20 microliters of different concentrations of compound or vehicle alone. Each treatment was done in triplicate. After 48 hours of incubation at 37°C and 5% CO₂, 20 µL of MTT solution was added (5 mg/mL of 1x PBS), followed by further incubation for 4 hours and 30 minutes at 37°C and 5% CO₂. During these 4 hours and 30 minutes, MTT (yellowish) is metabolized to formazan salt (bluish in color) by living cells. After this, 100 µL sodium dodecyl sulphate (SDS) 10% + 0.1M of HCl were added to dissolve the formazan crystals. After incubating overnight, absorbance reading at 570 nm was carried out. The percentage of cells surviving the treatment were calculated relative to the number of surviving cells in the medium without addition of the compounds in question ("negative control").
c) Determination of IC: IC₅₀ is defined as the concentration of a compound at which 50% of maximal inhibition is obtained. After reading the absorbance, survival curves were constructed and the values of IC₅₀ were obtained with the GraphPad Prism software.

### RESULTS: ACTION OF ACIL HYDRAZONES AND OXADIAZOLES ON B AND T LINEAGE LEUKEMIA CELLS (HEK AND JURKAT RESPECTIVELY)

The cytotoxic effect of the synthesized compounds on human leukemia Jurkat and HEK cells was studied by cell viability assay (MTT) according to the methods described by Pieters *et al* (Pieters, R.; et al. In vitro drug sensitivity of cells from children with leukemia using the MTT assay with improved culture conditions. Blood, 1990, 76, 2327-2336; and Pieters, R.; et al. Relation of cellular drug resistance to long-term clinical outcome in childhood acute lymphoblastic leukaemia. Lancet, 1991, 338, 399-403) and Kaspers *et al* (Kaspers, G. J.; et al. In vitro cellular drug resistance and prognosis in newly diagnosed childhood acute lymphoblastic leukemia. Blood, 1997 90, 2723). The results are shown in Table 3.

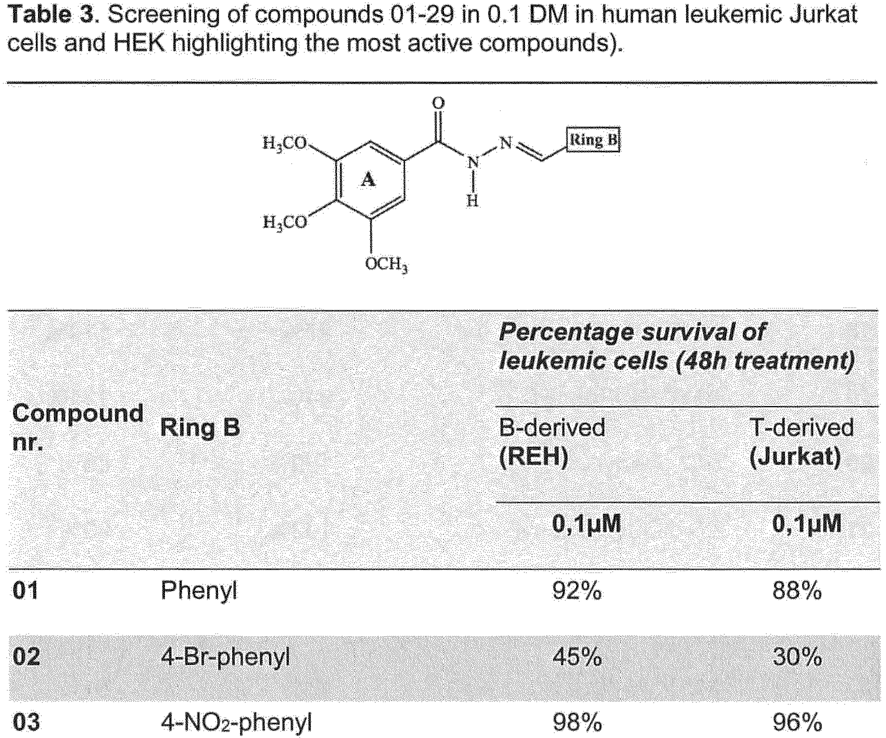

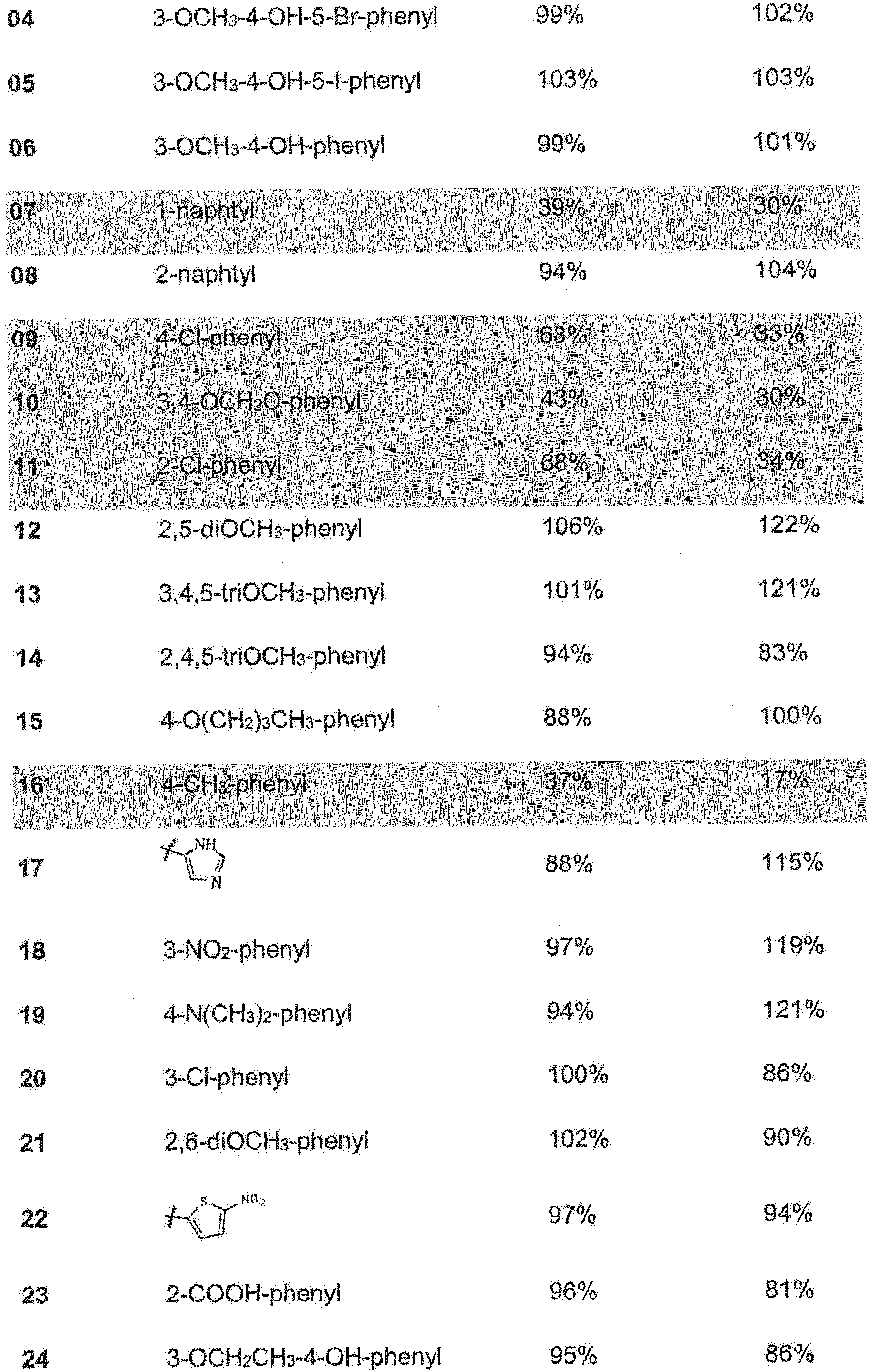

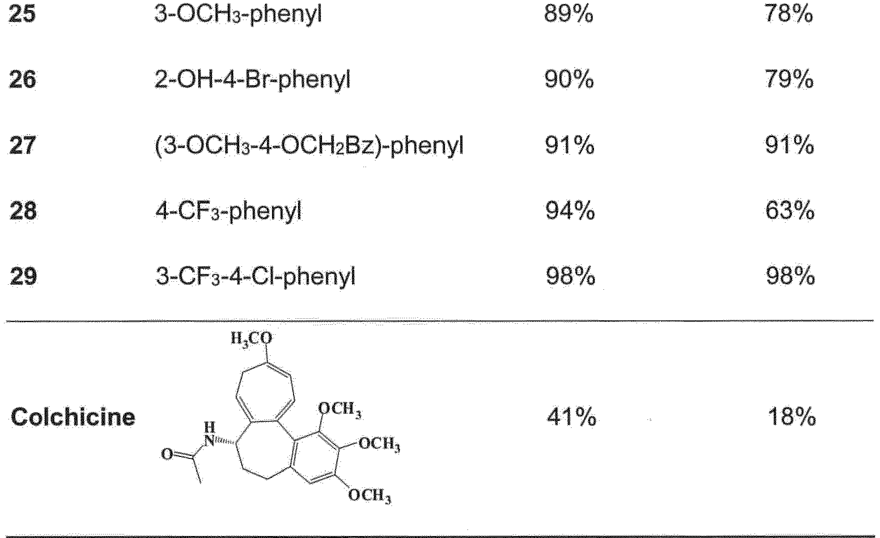

In order to analyze the influence of the methoxy groups in the A ring of acyl-hydrazones **01-29,** we tested the series of complementary acyl-hydrazones (**30-35**). None of the compounds in this series showed activity against cells in the screening performed, thus proving the necessity of the trimethoxylated ring for antileukemic activity.

In a third attempt, the search for compounds correlated to the active ones, the cyclic derivatives 1,3,4-oxadiazoles (**36-38**) of 3,4,5-trimethoxyphenyl-hydrazones were tested. Similarly to the complementary acyl-hydrazones (**30-35**), none of the 1,3,4-oxadiazoles (**36-38**) was shown to be active. The explanation of these results may be due to the rigidity of the rings promoted by the cyclization, preventing the interaction of the molecule with the target.

Thus, the compounds **02, 07, 09, 10, 11** and **16** were selected to have their IC₅₀ determined; values are presented in Table 4.

**Table 4. IC₅₀ of the most active compounds in leukemic Jurkat cells and REH.**

| | | | |
|---|---|---|---|
| 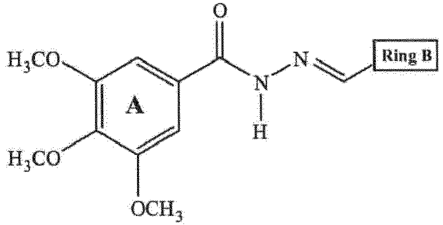 | | | |

| **Compound nr.** | **Ring B** | ***IC₅₀ (nM)- 48h of treatment*** | |
|---|---|---|---|
| | | B **(REH)** | T **(Jurkat)** |
| **02** | 4-Br-phenyl | 33.7 | 31.4 |
| **07** | 1-naphtyl | 25.4 | 15.7 |
| **09** | 4-Cl-phenyl | 328.3 | 202.9 |
| **10** | 3,4-OCH₂O-phenyl | 206.2 | 154.5 |
| **11** | 2-Cl-phenyl | 458.7 | 189.4 |
| **16** | 4-CH₃-phenyl | 70.6 | 71.5 |
| **Colchicine** | 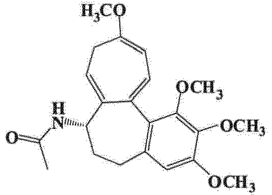 | 9.5 | 7.7 |

The acyl-hydrazones **02** and **07** showed excellent results, with an IC₅₀ of 33.7nM and 25.4nM for the leukemic strain REH and 31.4nM and 15.7nM for Jurkat.

### Example 6 Experiments for determining the action mechanism of the compounds with DNA microarrays:

The cells were incubated for 12 hours in fresh medium before the experiments. Two million cells from the leukemic cell line HL60, in triplicate, were treated with a 125 nM dose of compound **07** or with dimethylsulfoxide (DMSO) vehicle for 6 h, in DMEM supplemented with 10% fetal bovine serum. At the end of this period, the cells were recovered by brief centrifugation and lysed in a solution of guanidine of the *RNAspin Mini RNA Isolation* kit (GE).
a) Purification of RNA and preparation of the biotinylated probe: Total RNA was extracted from cells using the *RNAspin Mini RNA Isolation* kit (GE) following the manufacturer's instructions. A total of 100 ng of RNA was used to prepare biotinylated complementary RNA probes (Bio-cRNA), by cDNA synthesis followed by amplification by *in vitro* transcription, using the *GeneChip WT cDNA Synthesis and Amplification* kit (Affymetrix) according to the recommendations of the manufacturer.
b) Oligonucleotide microarrays and hybridization arrays: the cRNA probe of each replica was hybridized in an oligonucleotide microarray of the human genome *GeneChip Human Gene 1.0 ST Array* (Affymetrix). Hybridization and subsequent washes and development of microarrays were performed following manufacturer's recommendations.
c) Data analysis: the results of the microarray hybridization were read in the Affymetrix Gene Chip Scanner 3000-7G. Data were analyzed in the Bioconductor platform. To obtain the values of gene expression, we used the iterPLIER+16 algorithm at the gene level with the Affymetrix Expression Console. Genes differentially expressed in response to compound 07 were obtained by regression analysis using the LIMMA package and the Fold Change (FC) criteria > 1.5 and p <0.05. The list of differentially expressed genes were analyzed using the CMap (http://www.broad.mit.edu/cmap/) for identification of possible action mechanisms related to the action of known compounds (Lamb, J., Crawford E.D. The Connectivity Map: Using Gene-Expression Signatures to Connect Small Molecules, Genes and Disease Science 2006, 313, 1929-1935).

To establish whether a given gene set components (gene set) are co-regulated in the microarray experimental data, a Gene Set Expression Analysis (GSEA; http://www.broadinstitute.org/gsea/) was used. The GSEA algorithm creates a list of genes represented on the microarrays, arranged with differential expression between the two experimental groups (treated with compound **07** and control). In this case, the genes that are suppressed by compound **07** are at the top of the list, while genes that are induced are at the bottom. The algorithm will then find in this list the genes that make up a certain gene pool. If these genes are significantly over-represented at the top of this list, it can be said that the whole gene is repressed by the compound **07.** In the converse situation, the whole gene is induced by drug treatment. The gene sets analyzed by GSEA were obtained from several public databases (BioCarta, Signaling Pathway Database, Signaling Gateway, Signal Transduction Knowledge Environment, Human Protein Reference Database, GenMAPP, KEGG, Gene Ontology, Sigma-Aldrich Pathways, Gene Arrays BioScience Corp., Human Cancer Genome Anatomy Consortium and NetAffx). The mean expression values of probe sets related to the same gene were considered, using 1000 permutations as the setting of the False Discovery Rate (FDR) q-value. Gene sets with less than 5 and more than 500 components were not considered.

### RESULTS: ACTION MECHANISM PROPOSED BY STUDIES WITH DNA MICROARRAYS

In order to determine the action mechanism of these compounds, we selected compound **07** for analysis of gene expression through a DNA biochip. These trials have generated a dataset (algorithms) that were subsequently interpreted by Connectivity Maps (CMap) (www.broadinstitute.org/cmap) and GSEA analysis.

Treatment of HL-60 cells with compound **07** resulted in transcription repression of 102 genes and in the transcription activation of 353 genes (FC> 1.5, p <0.05).

The results obtained by the analysis of microarray gene expression, of the cells treated with the test compound **07,** were analyzed in CMap. Genes differentially expressed in cells under the action of compound **07** were cross linked with a series of gene lists responsive to different drugs. These lists are part of the CMap database. When taken together, the drug data grouped by the ATC system (Anatomical Therapeutic Chemical; http://www.whocc.no/atcddd/), the effect of compound **07** was most similar to the effect of the drugs of the P02CA group, that are Benzimidazole derivatives (e.g. albendazole, mebendazole, fenbendazole, thiabendazole) used as anthelmintics. The results of the analysis of compound **07** in CMap are shown in Table 5a below.

The action mechanism of these drugs is through the inhibition of tubulin and have been recently described as potential candidates for the treatment of leukemias (Spagnuolo PA, et al, The antihelmintic flubendazole inhibits microtubule function through a mechanism distinct from Vinca alkaloids and displays preclinical activity in leukemia and myeloma. Blood 2010; 115(23):4824-33) and solid tumors (Doudican N, et al, Mebendazole induces apoptosis via Bcl-2 inactivation in chemoresistant melanoma cells. Mol Cancer Res 2008; 6(8):1308-315; Gupta K, et al; antifungal antimitotic compound benomyl inhibits brain microtubule polymerization and dynamics and cancer cell proliferation at mitosis, by binding to a novel site in tubulin. Biochemistry 2004; 43(21):6645-6655).

**Table 5a: Compounds positively correlated with the compound 07 by ATC code.**

| Rank | ATC Code | Average | n | Enrichment | p | Specificity | Non-null % |
|---|---|---|---|---|---|---|---|
| 1 | P02CA | 0.404 | 16 | 0.511 | 0.00018 | 0.017 | 68 |
| 2 | C01AA | 0.541 | 10 | 0.63 | 0.00022 | 0.1852 | 80 |
| 3 | N05AC | 0.32 | 24 | 0.42 | 0.00026 | 0.2477 | 62 |
| 4 | P01AX | 0.415 | 9 | 0.638 | 0.00048 | 0.0194 | 77 |
| 5 | V03AF | -0.532 | 5 | -0.774 | 0.00106 | 0.0153 | 80 |
| 6 | P02CX | 0.585 | 6 | 0.724 | 0.00117 | 0.0884 | 83 |
| 7 | D06BX | -0.386 | 5 | -0.768 | 0.00124 | 0 | 60 |
| 8 | G04BD | 0.354 | 8 | 0.597 | 0.00279 | 0 | 62 |
| 9 | S01GA | 0.257 | 20 | 0.373 | 0.0056 | 0.0148 | 60 |
| 10 | G04CB | -0.314 | 6 | -0.642 | 0.00612 | 0.1281 | 50 |
| 11 | P02CE | 0.53 | 4 | 0.746 | 0.0079 | 0 | 100 |
| 12 | S01EX | 0.567 | 3 | 0.84 | 0.00803 | 0 | 100 |
| 13 | P02BX | 0.513 | 4 | 0.733 | 0.00985 | 0 | 0 |
| 14 | G03CC | 0.352 | 17 | 0.38 | 0.01053 | 0.05 | 64 |
| 15 | P03AA | 0.47 | 5 | 0.666 | 0.01093 | 0.1214 | 80 |
| 16 | N05CE | -0.294 | 4 | -0.708 | 0.01486 | 0.0423 | 50 |
| 17 | R01AA | 0.248 | 16 | 0.376 | 0.01544 | 0.016 | 56 |
| 18 | R01AB | 0.248 | 16 | 0.376 | 0.01544 | 0.016 | 56 |
| 19 | D08AC | -0.455 | 5 | -0.639 | 0.01568 | 0.0376 | 80 |
| 20 | R02AA | -0.455 | 5 | -0.639 | 0.01568 | 0.0376 | 80 |

Compared to data of other *perturbations* that are part of the CMap platform, considering only data for the HL-60 cell line, the effect of compound **07** was most similar to the effect of tanespimycin (enrichment = 0.699, p <0.001), which is a drug that inhibits Hsp90. Hsp90 is a chaperone that interacts and collocates with tubulin (C Garnier, et al, Heat-shock protein 90 (hsp90) binds in vitro to tubulin dimer and inhibits microtubule formation. Biochem Biophys Res Commun 1998, 250(2):414-9; Redmond T et al; Immunofluorescence colocalization of the 90-kDa heat-shock protein and microtubules in interphase and mitotic mammalian cells. Eur J Celi Biol 1989; 50(1):66-75; Sanchez ER et al; Evidence that the 90-kilodalton heat shock protein is associated with tubulin-containing complexes in L cell cytosol and in intact PtK cells. Mol Endocrinol 1988; 2(8):756-60; Czar MJ et al; Immunofluorescence localization of the 90-kDa heat-shock protein to cytoskeleton Eur J Cell Biol 1996;. 70 (4) :322-30), protecting it from denaturation and keeping it in a compatible for polymerization (F Weis et al state, The 90-kDa heat tubulin shock protein Hsp90 protects against thermal denaturation. J Biol Chem 2010; 285(13):9525-34). The result suggests that, like tanespimycin, compound **07** can affect the process of assembling tubulin or destabilize the already formed microtubules.

**Table 5b: Compound positively correlated with compound 07, for cell line.**

| Rank | ATC Code | Average | n | Enrichment | p | Specificity | Non-null % |
|---|---|---|---|---|---|---|---|
| 1 | geldanamycin - MCF7 | 0.598 | 10 | 0.781 | 0 | 0.0168 | 90 |
| 2 | tanespimycin - MCF7 | 0.551 | 36 | 0.676 | 0 | 0.0263 | 80 |
| 3 | trichostatin A - MCF7 | -0.322 | 92 | -0.396 | 0 | 0.5347 | 55 |
| 4 | trichostatin A - PC3 | -0.275 | 55 | -0.385 | 0 | 0.5058 | 50 |
| 5 | tanespimycin - HL60 | 0.571 | 12 | 0.699 | 0.00002 | 0.0814 | 75 |
| 6 | methotrexate - HL60 | -0,835 | 2 | -0,989 | 0,00026 | 0 | 100 |
| 7 | helveticoside - PC3 | 0.8 | 2 | 0.987 | 0.00032 | 0.0128 | 100 |
| 8 | alvespimycin - MCF7 | 0.624 | 7 | 0.703 | 0.0005 | 0.0326 | 85 |
| 9 | 0175029-0000 - PC3 | 0,598 | 4 | 0,856 | 0,00056 | 0,0573 | 100 |
| 10 | PNU-0251126 - PC3 | -0,57 | 4 | -0,868 | 0,0006 | 0,0067 | 100 |
| 11 | puromycin - MCF7 | 0.763 | 2 | 0.98 | 0.0007 | 0.0699 | 0 |
| 12 | monorden - MCF7 | 0.414 | 12 | 0.534 | 0.0009 | 0.0909 | 58 |
| 13 | finasteride - MCF7 | -0.628 | 3 | -0.918 | 0.00096 | 0.019 | 100 |
| 14 | methyldopate - MCF7 | -0.726 | 2 | -0.978 | 0.00107 | 0.008 | 100 |
| 15 | albendazole - MCF7 | 0.765 | 2 | 0.964 | 0.00225 | 0 | 100 |
| 16 | PHA-00745360 - CF7 | -0.374 | 4 | -0.812 | 0.00229 | 0.0073 | 75 |
| 17 | bendroflumethiazide - MCF7 | 0.636 | 3 | 0.894 | 0.00234 | 0 | 100 |
| 18 | 0198306-0000 - MCF7 | -0,688 | 2 | -0,964 | 0,00288 | 0 | 100 |
| 19 | tanespimycin - PC3 | 0.41 | 12 | 0.496 | 0.00311 | 0.2159 | 66 |
| 20 | CP-690334-01 - PC3 | -0,431 | 4 | -0,799 | 0,00318 | 0,0362 | 75 |

The dynamics of cellular processes demand continued cytoskeletal reorganization, therefore, the polymerisation and depolymerisation of tubulin. Tubulin inhibitors can act in two ways: (1) inhibiting tubulin polymerization or (2) stabilizing tubulin in such a way as to inhibit depolymerization. GSEA analysis showed that the compound **07** causes drastic reduction of the expression of a set of genes of tubulin and tubulin-specific chaperones (Figure 1), which is typical of compounds which inhibit tubulin polymerization. Inhibition of tubulin polymerization results in accumulation of free tubulin (non polymerized) in the cell cytoplasm. The free cytoplasmic tubulin negatively autoregulates tubulin mRNA expression by suppressing the formation of new tubulin mRNA and accelerating the degradation of the existing mRNA (Caron JM, et al; autoregulation of tubulin synthesis in hepatocytes and fibroblasts. Celi J Biol 1985; 101:1763-72). In contrast, compounds which stabilize tubulin filaments lead to increased expression of tubulin.

### Example 7. Effect of compound 07 on the progression of cells through the cell cycle, activation of regulators of the cell cycle and induction of apoptosis:

The cells were incubated for 12 hours in fresh medium before experiments. Two million cells from the Jurkat line were incubated for 12h, 18h, 24h and 30h in RPMI-640 medium supplemented with 10% Fetal Bovine Serum (FCS) and 0.2% Penicillin / Streptomycin (PS) with a concentration of 125 nM of compound **07** or DMSO (vehicle).
a) Screening assays of the cell cycle: The cells treated or not with compound **07** were fixed in 70% ethanol for 2 hours, washed in PBS and incubated for 15 min at 37°C in 1 mL of Triton X-100 0.1% 0.2 mg/mL of RNAse and 20 µg/mL propidium iodide in PBS. 20,000 events were collected on FACSCalibur flow cytometer with red fluorescence quantification, excluding any cell aggregates by the standard width vs. red fluorescence peak area. The data deconvolution to obtain the percentage of cells in each phase of cell cycle was performed using ModFit software v2.0.
b) Assays for quantification of apoptosis with Annexin V/PI labeling: The cells treated or not with compound **07** were rinsed in PBS and labeled with Annexin V Apoptosis Assay kit (Invitrogen). Briefly, cells were resuspended in appropriate buffer containing calcium and incubated for 15 min with Annexin V-FITC and propidium iodide 5 µg/mL. 10,000 events were collected on FACSCalibur flow cytometer, excluding any debris by forward vs standard. side scatter, with quantification of green and red fluorescence.
c) Western Blot testing for analysis of expression and phosphorylation of cell cycle regulatory proteins. The cells treated or not with compound **07** were lysed in buffer containing 50 mM Tris pH 7.7, 150 mM NaCl, 5 mM EDTA, 1% Sigma phosphatase inhibitor cocktail I, 1% Sigma phosphatase inhibitor cocktail II, 1% Sigma protease inhibitor cocktail II, 1% Igepal, 0.1% SDS, 0.5% sodium deoxycholate and 1 mM PMSF. After incubation for 30 min on ice and 3 cycles of freezing and thawing, extracts were centrifuged at 10,000 rpm for 20 min at 4°C to sediment membrane debris. Of the supernatant, we proceeded with protein quantification by Bradford reagent (Biorad). The samples were boiled for 5 min with prior addition of β-mercaptoethanol and 100 µg of protein were subjected to acrylamide:bisacrylamide gel electrophoresis (39:1) 10% with SDS and electrotransferred to 0.45 µm nitrocellulose membrane (Schleicher & Schuell). Immunodetection was performed by incubation of the membrane with *Cell Cycle* / *Checkpoint Antibody Sampler kit* # *9917* antibodies (Cell Signaling Technology) and *Cyclin Dependent Kinase Inhibitor Antibody Sampler kit* # 9867 (Cell Signaling Technology) following the manufacturer's instructions. The development of the results was performed with the *Super Signal West Pico Chemiluminiscent Substrate kit* (Pierce) followed by exposure to X-ray film.

### RESULTS: COMPOUND 07 INDUCES CELL CYCLE ARREST IN G2 AND CELL DEATH BY APOPTOSIS

Results consistent with the inhibition of tubulin were obtained in the cell cycle analysis of Jurkat cells that were treated with compound **07.** Jurkat cells treated with compound **07** showed cell cycle arrest in G2/M (Figure 2).

Changes in the expression and phosphorylation state of various proteins that regulate the cell cycle were investigated. As shown in Figure 3, after 12 hours of treatment with compound **07,** it is possible to detect the phosphorylation of Chk2 in Thr68. Phosphorylation of the Thr68 residue is done by the *Ataxia Telangiectasia Mutated* factor (ATM) and results in activation of Chk2 and subsequent phosphorylation of a number of downstream targets, including cdc25, BRCA1, p53 and E2F, which are important control factors (chekpoints) of the cell cycle, repair of DNA damage and induction of apoptosis in response to DNA damage (Falck J, et al, The ATM-Chk2-Cdc25A checkpoint pathway guards against radioresistant DNA synthesis. Nature 2001, 410(6830): 842-7; Matsuoka S, et al; Linkage of ATM to cell cycle regulation by the Chk2 protein kinase. Science 1998, 282(5395): 1893-7; Lee JS et al; hCdsl-mediated phosphorylation of BRCA1 regulates the DNA damage response. Nature 2000: 404(6774) 201-4; Hirao A et al, DNA damage-induced activation of p53 by the checkpoint kinase Chk2. Science 2000, 287(5459): 1824-7; C Stevens et al, Chk2 activates E2F-1 in response to DNA damage. Nat Cell Biol 2003: 5(5): 401-9). The absence of detectable p53 results in the western blot (Figure 3) can be explained by the low p53 expression in Jurkat cells (Vigorito E, et al;. Contributions of p53 and PMA to gamma-irradiation induced apoptosis in Jurkat cells. Hematol Cell Ther 1999, 41(4):153-61).

Treatment with Compound **07** resulted in the repression of the expression of CDK4 and to a lesser extent also of CDK6, shorter isoform of CDK2 and larger isoform of CDK9 (Figure 3). D/CDK4/CDK6 cyclin and cyclin E/cdk2 complexes phosphorylate and inactivate Rb to allow cell cycle progression. The decrease in CDK2, CDK4 and CDK6 in cells treated with compound **07** decreases inactivation of Rb, which is then able to perform its duties of the inhibition of cell cycle progression and induce apoptosis.

**Example 8. Effect of compound 07 on healthy human lymphocytes and on colony formation by evaluating its selectivity:** *a) in vitro assays of sensitivity* / *resistance of the cells to the compound **07** by the MTT method:* for the test of compound **07** against normal lymphocytes, blood from human donors was separated in a *ficoll* gradient and the mononuclear cells were cultivated at 200,000 cells/well, in 80 microliters of RPMI-1640 (containing 10% FBS, 100 IU/mL penicillin and 100 µg/mL streptomycin) and 20 microliters of phytohemagglutinin solution (Cultilab) in each well for stimulation of T lymphocytes. Compound **07** was added in serial dilutions (50%) from 300 to 1 nm. As a control, parallel tests were run with cells from leukemic Jurkat and REH cell lines, and experiments with colchicine with the same concentrations as compound 07. Cytotoxicity was assessed after 48 hours by the MTT method, as described above. b) *Colony formation experiments:* Cytotoxicity of compound **07** was also evaluated with a colony formation assay of hematopoietic cells. A "HSC-CFU complete with Epo" kit from Miltenyi (Cat # 130-091-280) was used following the manufacturer's recommendations. Bone marrow cells from healthy donors were cultured in semi-solid methylcellulose medium supplemented with bovine fetal serum, bovine serum albumin, different growth factors (GM-CSF, G-CSF, SCF, IL-3, IL-6, Epo) and compound **07,** whose action on hematopoiesis was evaluated. After two weeks of culture at 37°C and 5% CO₂, an assessment of the number of granulocytes (CFU-G), macrophage (CFU-M), granulocyte / macrophage (CFU-GM), erythroid (BFU -E and CFU-E) and mixed (CFU-GEMM) colonies was carried out.

### RESULTS: SELECTIVITY OF COMPOUND 07

Compound **07** was tested on healthy and mature T lymphocytes stimulated with phytohemagglutinin to assess its action against normal cells. Colchicine was used as a positive control. The results are shown in Table 6.

**Table 6. Percentage of survival in healthy and phytohemagglutinin-stimulated lymphocytes treated with different concentrations of compound 07.**

| **Compound nr.** | **Concentration (nM)** | | | | | | | |
|---|---|---|---|---|---|---|---|---|
| | **300** | **150** | **75** | **37.5** | **18.75** | **9.38** | **4.6** | **0** |
| **07** | 95% | 107% | 108% | 101% | 105% | 105% | 103% | 100% |
| **Colchicine** | 63% | 69% | 75% | 84% | 95% | 110% | 109% | 100% |

At concentrations of 300 nM compound 07 had a toxicity of 5% relative to the control, whereas colchicine at the same concentration caused 37% inhibition on the viability of lymphocytes T.

A dose-response analysis of compound **07** against leukemic cells as compared to normal lymphocytes stimulated with phytohemagglutinin (Figure 5) shows that concentrations of up to 10 times higher than the cytotoxic dose for leukemic cells do not appear to affect normal lymphocytes induced by phytohemagglutinin. This indicates that compound **07** could act in leukemic cells without affecting normal immune function of patients.

To determine the effect of compound 07 in hematopoiesis, the same assay was tested on colony formation of bone marrow cells cultured in semi-solid methylcellulose medium plus growth factors. As shown in Table 7, compound 07 at concentrations very close to IC₅₀ (20 nM), displays inhibitory activity on erythrocyte formation comparable to a PI3K inhibitor, used in the assay as a positive control. Compound 07 also has an inhibitory activity against granulocytes and macrophages, although low in intensity when compared to a P'I3K inhibitor. At a 200 nM concentration, corresponding to 10 times the IC₅₀ for average LLA lineages, compound 07 completely inhibited hematopoiesis.

**Table 7. Colony formation of bone marrow cells (%) using the compound 07.**

| | BFU-E | CFU-E | CFU-G | CFU-M | CFU-GM | CFU-GEMM |
|---|---|---|---|---|---|---|
| Negative control | 30.7±13.9 | 27.0±9.5 | 20.0±4.0 | 40.7±5.1 | 46.7±14.6 | 5.7±7.4 |
| 2 nM compound 7 | 39 | 23 | 11 | 17 | 52 | 1 |
| 20 nM compound 7 | 26 | 11 | 14 | 25 | 27 | 2 |
| 200 nM compound 7 | 0 | 0 | 0 | 0 | 0 | 0 |
| Pi3K inhibitor | 18 | 12 | 8 | 9 | 20 | 1 |

| | | | | | | |
|---|---|---|---|---|---|---|
| * For control the average ± standard deviation of 3 replicates are presented. | | | | | | |

As reported above, five novel 3,4,5-trimethoxyphenyl-hydrazones and three novel 3,4-oxadiazoles were obtained. All acyl-hydrazones and oxadiazoles synthesized and included within the scope of the present invention were evaluated in leukemic cells of Jurkat and HEK strains, and the compounds **02** and **07** showed excellent antileukemic activity, similar to the standard compound (colchicine). The action mechanism of compound **07** was determined using DNA microarrays, showing its inhibitory activity of tubulin, with Chk2 activation and Rb, cell cycle arrest in G2/M and induction of cell death by apoptosis. Subsequent tests showed the selectivity of the compound 07 for leukemic cells in the order of 10 times, when compared to its action in healthy human lymphocytes.

The 3,4,5-trimethoxyphenyl-hydrazones and related compounds and their analogs, comprising the oxadiazoles, present in this invention, therefore have great potential as drugs prototypes, pre-drugs, or drugs, for treatment of different diseases associated with cell proliferation such as leukemias, including acute lymphoblastic leukemia (ALL), tumors and inflammation.

The description of the present invention has been presented for purposes of illustration, and detail for future applications. However, the present description has no limiting character regarding the inventions described and exemplified as disclosed herein. Changes and modifications compatible with the described above, and the skill or knowledge of the relevant art, are within the scope of the present invention. It's a legitimate intent that the invention comprises in its scope all modifications and variations of the same, according to the report description and claims.

## Claims

1. A compound of formula I 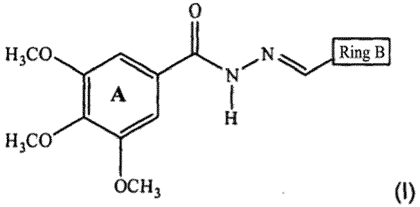 wherein Ring B is a substituent selected from the group comprising 3-OCH₂CH₃-4-OH-phenyl; and 2-OH-4-Br-phenyl.

2. A compound of Formula I 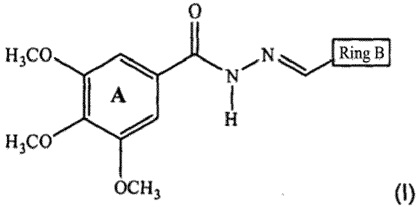 wherein the Ring B represents a substituent selected from the group comprising phenyl; 4-Br-phenyl; 4-NO₂-phenyl; 3-OCH₃-phenyl; 1-naphthyl; 2-naphthyl; 4-Cl-phenyl; 3-OCH₂CH₃-4-OH-phenyl; and 2-OH-4-Br-phenyl for use in treating cancer.

3. A compound for use in treating cancer according to Claim 2 wherein the Ring B represents a substituent selected from the group comprising phenyl; 4-Br-phenyl; 4-NO₂-phenyl; 3-OCH₃-phenyl; 1-naphthyl; 4-Cl-phenyl; 3-OCH₂CH₃-4-OH-phenyl; and 2-OH-4-Br-phenyl.

4. A pharmaceutical composition comprising a compound according to claim 1 and a pharmaceutically acceptable excipient.

5. A pharmaceutical composition for use in treating cancer comprising a compound as defined in claim 2 and a pharmaceutically acceptable excipient.

6. A compound according to claim 1 or composition according to claim 4 for use in treating a disease associated with cellular proliferation, wherein the disease associated with cell proliferation is selected from acute lymphoblastic leukemia (ALL), tumors and inflammation.

7. A compound of formula Ia 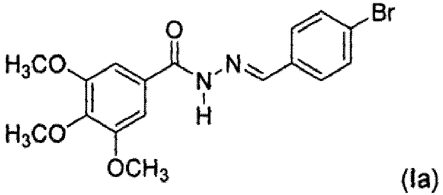 for use in the treatment of a disease associated with cell proliferation, wherein the disease associated with cell proliferation is selected from acute lymphoblastic leukemia (ALL), tumors and inflammation.

8. A compound of formula IIa 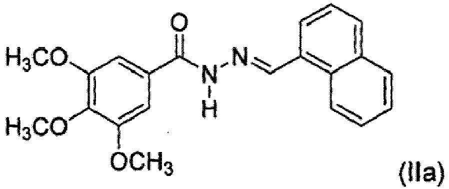 for use in the treatment of a disease associated with cell proliferation, wherein the disease associated with cell proliferation is selected from acute lymphoblastic leukemia (ALL), tumors and inflammation.

## Patentansprüche

1. Verbindung mit der Formel I: 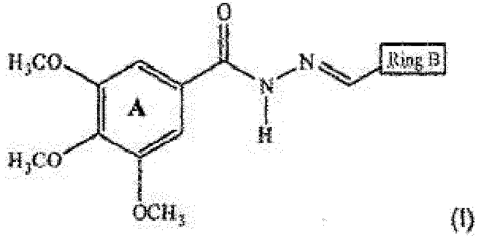 wobei Ring B ein Substituent ist, ausgewählt aus der Gruppe umfassend 3-OCH₂CH₃-4-OH-Phenyl; und 2-OH-4-Br-Phenyl.

2. Verbindung mit der Formel I: 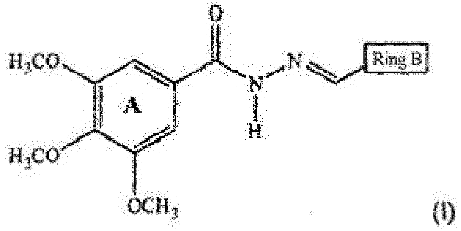 wobei der Ring B einen Substituenten darstellt, ausgewählt aus der Gruppe umfassend Phenyl; 4-Br-Phenyl; 4-NO₂-Phenyl; 3-OCH₃-Phenyl; 1-Naphthyl; 2-Naphthyl; 4-Cl-Phenyl; 3-OCH₂CH₃-4-OH-Phenyl; und 2-OH-4-Br-Phenyl für den Gebrauch beim Behandeln von Krebs.

3. Verbindung für den Gebrauch beim Behandeln von Krebs nach Anspruch 2, wobei der Ring B einen Substituenten darstellt, ausgewählt aus der Gruppe umfassend Phenyl; 4-Br-Phenyl; 4-NO₂-Phenyl; 3-OCH₃-Phenyl; 1-Naphthyl; 4-Cl-Phenyl; 3-OCH₂CH₃-4-OH-Phenyl; und 2-OH-4-Br-Phenyl.

4. Pharmazeutische Zusammensetzung, umfassend eine Verbindung nach Anspruch 1 und einen pharmazeutisch unbedenklichen Hilfsstoff.

5. Pharmazeutische Zusammensetzung für den Gebrauch beim Behandeln von Krebs, umfassend eine Verbindung nach Anspruch 2 und einen pharmazeutisch unbedenklichen Hilfsstoff.

6. Verbindung nach Anspruch 1 oder Zusammensetzung nach Anspruch 4 für den Gebrauch beim Behandeln einer Krankheit in Verbindung mit Zellproliferation, wobei die mit Zellproliferation verbundene Krankheit ausgewählt ist aus akuter lymphatischer Leukämie (ALL), Tumoren und Entzündung.

7. Verbindung mit der Formel la: 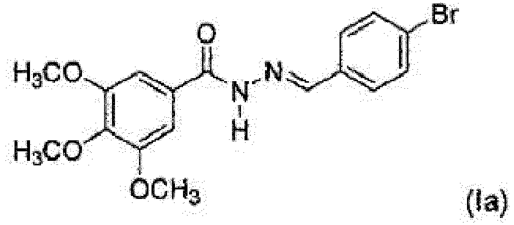 für den Gebrauch beim Behandeln einer Krankheit in Verbindung mit Zellproliferation, wobei die mit Zellproliferation verbundene Krankheit ausgewählt ist aus akuter lymphatischer Leukämie (ALL), Tumoren und Entzündung.

8. Verbindung mit der Formel IIa: 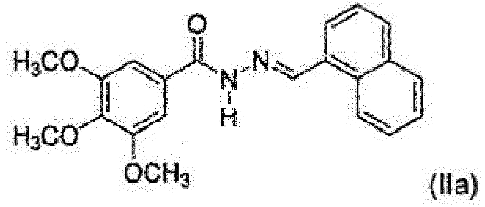 für den Gebrauch beim Behandeln einer Krankheit in Verbindung mit Zellproliferation, wobei die mit Zellproliferation verbundene Krankheit ausgewählt ist aus akuter lymphatischer Leukämie (ALL), Tumoren und Entzündung.

## Revendications

1. Composé de formule I 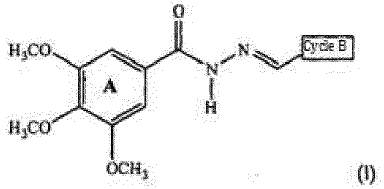 dans lequel le cycle B est un substituant choisi dans le groupe constitué par le 3-OCH₂CH₃-4-OH-phényle ; et le 2-OH-4-Br-phényle.

2. Composé de formule I 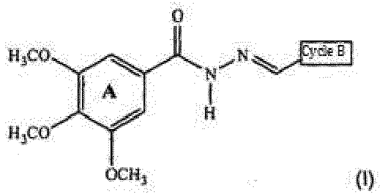 dans lequel le cycle B représente un substituant choisi dans le groupe constitué par le phényle ; le 4-Br-phényle ; le 4-NO₂-phényle ; le 3-OCH₃-phényle ; le 1-naphtyle ; le 2-naphtyle ; le 4-Cl-phényle ; le 3-OCH₂CH₃-4-OH-phényle ; et le 2-OH-4-Br-phényle pour une utilisation dans le traitement du cancer.

3. Composé pour une utilisation dans le traitement du cancer selon la revendication 2, dans lequel le cycle B représente un substituant choisi dans le groupe constitué par le phényle ; le 4-Br-phényle ; le 4-NO₂-phényle ; le 3-OCH₃-phényle ; le 1-naphtyle ; le 4-Cl-phényle ; le 3-OCH₂CH₃-4-OH-phényle ; et le 2-OH-4-Br-phényle.

4. Composition pharmaceutique comprenant un composé selon la revendication 1 et un excipient pharmaceutiquement acceptable.

5. Composition pharmaceutique pour une utilisation dans le traitement du cancer comprenant un composé selon la revendication 2 et un excipient pharmaceutiquement acceptable.

6. Composé selon la revendication 1 ou composition selon la revendication 4 pour une utilisation dans le traitement d'une maladie associée à la prolifération cellulaire, laquelle maladie associée à la prolifération cellulaire est choisie parmi la leucémie aiguë lymphoblastique (LAL), les tumeurs et l'inflammation.

7. Composé de formule la 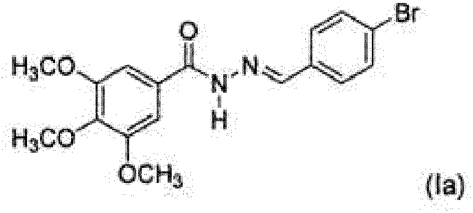 pour une utilisation dans le traitement d'une maladie associée à la prolifération cellulaire, laquelle maladie associée à la prolifération cellulaire est choisie parmi la leucémie aiguë lymphoblastique (LAL), les tumeurs et l'inflammation.

8. Composé de formule IIa 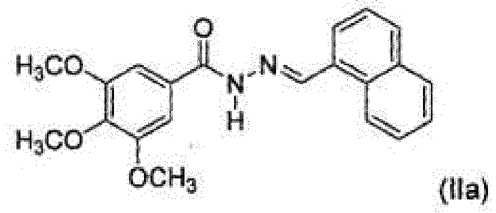 pour une utilisation dans le traitement d'une maladie associée à la prolifération cellulaire, laquelle maladie associée à la prolifération cellulaire est choisie parmi la leucémie aiguë lymphoblastique (LAL), les tumeurs et l'inflammation.
